(19) Europäisches Patentamt
European Patent Office
Office européen des brevets

(11) EP 3 929 181 A1

(12) **EUROPEAN PATENT APPLICATION**
published in accordance with Art. 153(4) EPC

(43) Date of publication:
**29.12.2021 Bulletin 2021/52**

(21) Application number: **20760294.7**

(22) Date of filing: **19.02.2020**

(51) Int Cl.:
*C07D 237/14* (2006.01) *A61K 31/50* (2006.01)
*A61P 29/00* (2006.01)

(86) International application number:
**PCT/CN2020/075790**

(87) International publication number:
**WO 2020/169042 (27.08.2020 Gazette 2020/35)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority:
**20.02.2019 CN 201910125750**
**09.05.2019 CN 201910384992**
**27.06.2019 CN 201910567035**
**09.01.2020 CN 202010020863**

(71) Applicants:
- **Jiangsu Hengrui Medicine Co., Ltd.**
  **Lianyungang, Jiangsu 222047 (CN)**
- **Shanghai Hengrui Pharmaceutical Co., Ltd.**
  **Shanghai 200245 (CN)**

(72) Inventors:
- **YANG, Fanglong**
  **Shanghai 200245 (CN)**
- **YU, Nan**
  **Shanghai 200245 (CN)**
- **CHI, Jiangtao**
  **Shanghai 200245 (CN)**
- **LIU, Zhiwei**
  **Shanghai 200245 (CN)**
- **HE, Feng**
  **Shanghai 200245 (CN)**
- **TAO, Weikang**
  **Shanghai 200245 (CN)**

(74) Representative: **Viganò, Elena et al**
**Dragotti & Associati S.r.l.**
**Via Nino Bixio, 7**
**20129 Milano (IT)**

# (54) 6-OXO-1,6-DIHYDROPYRIDAZINE PRODRUG DERIVATIVE, PREPARATION METHOD THEREFOR, AND APPLICATION THEREOF IN MEDICINE

(57) Specifically, the present invention relates to the 6-oxo-1,6-dihydropyridazine prodrug derivative shown in general formula (I), a preparation method therefor, a pharmaceutical composition containing the derivative, a use thereof as a NaV inhibitor, and a use thereof in the preparation of a drug for the treatment and/or prevention of pain and pain-related diseases.

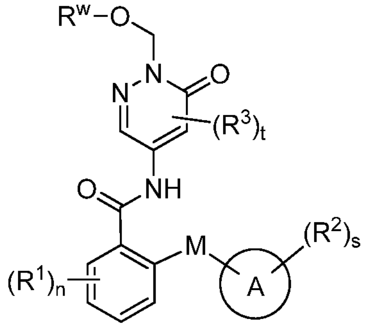

(I)

EP 3 929 181 A1

## Description

### TECHNICAL FIELD

The present disclosure belongs to the field of medicine, and relates to a 6-oxo-1,6-dihydropyridazine prodrug derivative, a method for preparing the same, and a use thereof in medicine. In particular, the present disclosure relates to a 6-oxo-1,6-dihydropyridazine prodrug derivative of formula (I), a method for preparing the same, a pharmaceutical composition comprising the same, a use thereof as a $Na_V$ inhibitor, and a use thereof in the preparation of a medicament for treating and/or alleviating pain and pain-related diseases.

### BACKGROUND

Pain is a complex physical and psychological activity, and is one of the most common clinical symptoms. International Association for the Study of Pain defines pain as "an unpleasant sensory and emotional experience associated with actual or potential tissue damage, which is a subjective feeling". Pain can act as a warning signal to remind the body to pay attention to potential dangers, and has an indispensable protective effect on the body's normal life activities. Moreover, pain is also a common clinical symptom. After the external stimulus that causes the pain disappears, the strong or persistent pain can lead to the disorder of the physiological function and seriously affect the quality of life of the living body. Statistics show that about one-fifth of people in the world suffer from moderate to severe chronic pain.

Pain originates from the nociceptors in the peripheral nervous system. The nociceptors are a kind of free nerve ending, and widely distributed in the skin, muscles, joints and visceral tissues of the whole body. The nociceptors can convert thermal, mechanical or chemical stimuli into nerve impulses (action potentials), transmit them to the cell body in the dorsal root ganglia (DRG) through the afferent nerve fibers and ultimately to the advanced nerve center, thereby causing pain. The generation and conduction of action potentials in neurons depend on the voltage-gated sodium channels ($Na_V$) located on the cytomembrane. When the cytomembrane is depolarized, the sodium ion channel is activated. The channel is opened, causing sodium ion influx, and further depolarizing the cytomembrane, resulting in the generation of action potentials. Therefore, the inhibition of abnormal sodium ion channel activity contributes to the treatment and alleviation of pain.

$Na_V$ is a kind of transmembrane ion channel protein. The protein consists of an alpha subunit with a molecular weight of 260 kD and a beta subunit with a molecular weight of 30-40 kD. According to the different alpha subunits, it can be divided into 9 subtypes, namely $Na_V1.1$ to $Na_V1.9$. Different subtypes show different tissue distribution and electrophysiological and pharmacological characteristics (Rush A.M., et al. J. Physiol. 2007, 579, 1-14.). According to whether it can be effectively inhibited by nanomolar tetrodotoxin (TTX), the sodium ion channels are divided into TTX-sensitive type (TTX-S) and TTX- resistant type (TTX-R). Among them, $Na_V1.1$, $Na_V1.2$, $Na_V1.3$ and $Na_V1.7$ are TTX-S type, and the coding genes thereof are located in human chromosome 2q23-24, and they are expressed in large amounts in neurons. $Na_V1.5$, $Na_V1.8$ and $Na_V1.9$ are TTX-R type, and the coding genes thereof are located in human chromosome 3p21-24. Among them, $Na_V1.5$ mainly exists in cardiomyocytes, and $Na_V$ 1.8 and $Na_V$ 1.9 exist in the peripheral nervous system (Goldin A.L., et al. Annu. Rev. Physiol. 2001, 63, 871-894.). $Na_V1.4$ and $Na_V1.6$ are TTX-S type, and exist in skeletal muscle and central nervous system in large amounts, respectively (Fozzard H.A., et al. Physiol. Rev. 1996, 76, 887-926.). The local anesthetic lidocaine relieves pain by inhibiting $Na_V$. Non-selective $Na_V$ inhibitors, such as lamotrigine, lacosamide and mexiletine have been successfully used to treat chronic pain.

$Na_V1.8$ is TTX-R type, the coding gene thereof is SCN10A. It mainly exists in trigeminal ganglion neurons and DRG neurons, and has the electrophysiological characteristics of slow inactivation and rapid recovery (Dib-Hajj S.D., et al. Annu. Rev. Neurosci. 2010, 33, 325-347.). In neurons expressing $Na_V1.8$, the rise of action potential is mainly composed of $Na_V1.8$ current. In some models for the study of neuropathic pain, nerve damage can increase the expression level of $Na_V1.8$ in axons and neuron cell bodies (Sleeper A.A., et al. J.Neurosci. 2000, 20, 7279-7289). The use of $Na_V1.8$ antisense oligonucleotide can significantly alleviate pain while reducing the expression of $Na_V1.8$ (Yoshimura N., et al. J.Neurosci. 2001, 21, 8690-8696). After carrageenan was injected into the paws of rats, the expression of $Na_V1.8$ in DRG neurons increased (Tanaka M., et al. G. NeuroReport 1998, 9, 967-972.). $Na_V1.8$-knockout mouse cannot show normal visceral inflammation pain (Kerr B.J., et al. NeuroReport 2001, 12, 3077-3080). After the human $Na_V1.8$ gene has a functional gain mutation, it will cause peripheral neuralgia (Faber C.G., et al. Proc. Natl. Acad. Sci. USA 2012, 109, 19444-19449.). According to a series of animal experiments and human genetic evidence, selective inhibition of $Na_V1.8$ has the potential to become a new type of analgesic therapy, which can be used for treating various types of pain such as inflammatory pain, neuropathic pain, postoperative pain and cancer pain.

The clinically used $Na_V$ inhibitors can inhibit sodium channels expressed in the heart and central nervous system due to lack of subtype selectivity. Therefore, the therapeutic window is narrow and the scope of application is limited. $Na_V1.8$ is mainly distributed in the peripheral nervous system, thus selective inhibition of $Na_V1.8$ can effectively reduce side effects. Therefore, it is necessary to develop $Na_V1.8$ inhibitors with higher activity, better selectivity, better pharmacokinetic properties and fewer side effects.

## SUMMARY OF THE INVENTION

The object of the present disclosure is to provide a compound of formula (I) or a tautomer, mesomer, racemate, enantiomer, diastereomer thereof, or mixture thereof, or a pharmaceutically acceptable salt thereof,

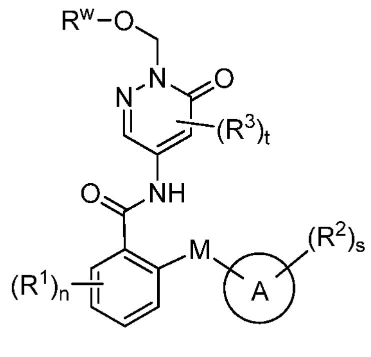

( I )

wherein:

M is selected from the group consisting of O atom, $CR^4R^5$ and S atom;
ring A is an aryl or heteroaryl;
each $R^1$ is identical or different and each is independently selected from the group consisting of hydrogen atom, halogen, alkyl, deuterated alkyl, alkoxy, deuterated alkoxy, haloalkyl, haloalkoxy, cyano, amino, nitro, hydroxy, hydroxyalkyl, cycloalkyl, heterocyclyl, aryl and heteroaryl;
each $R^2$ is identical or different and each is independently selected from the group consisting of hydrogen atom, halogen, alkyl, deuterated alkyl, alkoxy, deuterated alkoxy, haloalkyl, haloalkoxy, cyano, amino, nitro, hydroxy, hydroxyalkyl, cycloalkyl, cycloalkyloxy, heterocyclyl, aryl and heteroaryl, wherein the alkyl, cycloalkyl, heterocyclyl, aryl and heteroaryl are each optionally substituted by one or more substituents selected from the group consisting of alkyl, haloalkyl, halogen, amino, nitro, cyano, hydroxy, alkoxy, haloalkoxy, hydroxyalkyl, cycloalkyl, heterocyclyl, aryl and heteroaryl;
each $R^3$ is identical or different and each is independently selected from the group consisting of hydrogen atom, halogen, alkyl, alkoxy, haloalkyl, cyano, amino, nitro, hydroxy, hydroxyalkyl, cycloalkyl, heterocyclyl, aryl and heteroaryl;
$R^4$ and $R^5$ are identical or different and are each independently selected from the group consisting of hydrogen atom, deuterium atom, halogen, alkyl, alkoxy, haloalkyl, cyano, amino, nitro, hydroxy, hydroxyalkyl, cycloalkyl, heterocyclyl, aryl and heteroaryl;
$R^w$ is selected from the group consisting of hydrogen atom, alkyl, $-C(O)R^6$, $-S(O)_2OH$, $-S(O)_2O^-Q^+$, $-PO(OH)_2$, $-PO(OH)O^-Q^+$, $-PO(O^-)_22Q^+$ and $-PO(O^-)_2W^{2+}$; $Q^+$ is a pharmaceutically acceptable monovalent cation; $W^{2+}$ is a pharmaceutically acceptable divalent cation;
$R^6$ is selected from the group consisting of alkyl, alkoxy, alkenyl, carboxy and carboxylate, wherein the alkyl, alkoxy and alkenyl are each optionally substituted by one or more substituents selected from the group consisting of hydroxy, amino, carboxy and carboxylate;
n is 0, 1, 2, 3 or 4;
s is 0, 1, 2, 3 or 4; and
t is 0, 1 or 2.

The object of the present disclosure is to provide a compound of formula (I) or a tautomer, mesomer, racemate, enantiomer, diastereomer thereof, or mixture thereof, or a pharmaceutically acceptable salt thereof,

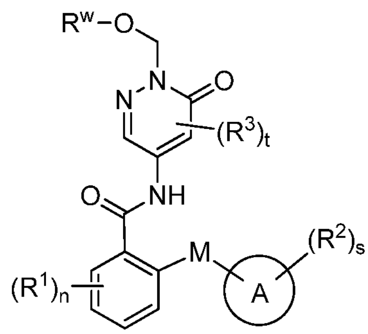

( I )

wherein:

M is selected from the group consisting of O atom, $CR^4R^5$ and S atom;
ring A is an aryl or heteroaryl;
each $R^1$ is identical or different and each is independently selected from the group consisting of hydrogen atom, halogen, alkyl, deuterated alkyl, alkoxy, deuterated alkoxy, haloalkyl, haloalkoxy, cyano, amino, nitro, hydroxy, hydroxyalkyl, cycloalkyl, heterocyclyl, aryl and heteroaryl;
each $R^2$ is identical or different and each is independently selected from the group consisting of hydrogen atom, halogen, alkyl, deuterated alkyl, alkoxy, deuterated alkoxy, haloalkyl, haloalkoxy, cyano, amino, nitro, hydroxy, hydroxyalkyl, cycloalkyl, cycloalkyloxy, heterocyclyl, aryl and heteroaryl, wherein the alkyl, cycloalkyl, heterocyclyl, aryl and heteroaryl are each optionally substituted by one or more substituents selected from the group consisting of alkyl, haloalkyl, halogen, amino, nitro, cyano, hydroxy, alkoxy, haloalkoxy, hydroxyalkyl, cycloalkyl, heterocyclyl, aryl and heteroaryl;
each $R^3$ is identical or different and each is independently selected from the group consisting of hydrogen atom, halogen, alkyl, alkoxy, haloalkyl, cyano, amino, nitro, hydroxy, hydroxyalkyl, cycloalkyl, heterocyclyl, aryl and heteroaryl;
$R^4$ and $R^5$ are identical or different and are each independently selected from the group consisting of hydrogen atom, deuterium atom, halogen, alkyl, alkoxy, haloalkyl, cyano, amino, nitro, hydroxy, hydroxyalkyl, cycloalkyl, heterocyclyl, aryl and heteroaryl;
$R^w$ is selected from the group consisting of hydrogen atom, alkyl, $-C(O)R^6$, $-S(O)_2OH$, $-S(O)_2O^-Q^+$, $-PO(OH)_2$, $-PO(OH)O^-Q^+$ and $-PO(O^-)_2W^{2+}$; $Q^+$ is a pharmaceutically acceptable monovalent cation; $W^{2+}$ is a pharmaceutically acceptable divalent cation;
$R^6$ is selected from the group consisting of alkyl, alkoxy, alkenyl, carboxy and carboxylate, wherein the alkyl, alkoxy and alkenyl are each optionally substituted by one or more substituents selected from the group consisting of hydroxy, amino, carboxy and carboxylate;
n is 0, 1, 2, 3 or 4;
s is 0, 1, 2, 3 or 4; and
t is 0, 1 or 2.

In some embodiments, the present disclosure provides a compound of formula (I) or a tautomer, mesomer, racemate, enantiomer, diastereomer thereof, or mixture thereof, or a pharmaceutically acceptable salt thereof,

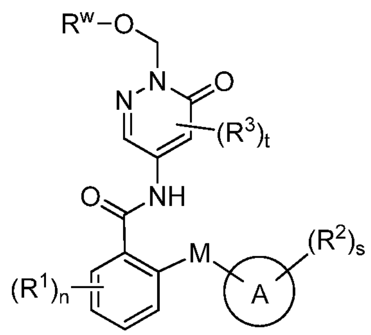

( I )

wherein:

M is selected from the group consisting of O, $CR^4R^5$ and S;
ring A is an aryl or heteroaryl;
each $R^1$ is identical or different and each is independently selected from the group consisting of hydrogen atom, halogen, alkyl, alkoxy, haloalkyl, haloalkoxy, cyano, amino, nitro, hydroxy, hydroxyalkyl, cycloalkyl, heterocyclyl, aryl and heteroaryl;
each $R^2$ is identical or different and each is independently selected from the group consisting of hydrogen atom, halogen, alkyl, alkoxy, haloalkyl, haloalkoxy, cyano, amino, nitro, hydroxy, hydroxyalkyl, cycloalkyl, cycloalkyloxy, heterocyclyl, aryl and heteroaryl, wherein the alkyl, cycloalkyl, cycloalkyloxy, heterocyclyl, aryl and heteroaryl are each optionally substituted by one or more substituents selected from the group consisting of alkyl, haloalkyl, halogen, amino, nitro, cyano, hydroxy, alkoxy, haloalkoxy, hydroxyalkyl, cycloalkyl, heterocyclyl, aryl and heteroaryl;
each $R^3$ is identical or different and each is independently selected from the group consisting of hydrogen atom, halogen, alkyl, alkoxy, haloalkyl, cyano, amino, nitro, hydroxy, hydroxyalkyl, cycloalkyl, heterocyclyl, aryl and heteroaryl;
$R^4$ and $R^5$ are identical or different and are each independently selected from the group consisting of hydrogen atom, halogen, alkyl, alkoxy, haloalkyl, cyano, amino, nitro, hydroxy, hydroxyalkyl, cycloalkyl, heterocyclyl, aryl and heteroaryl;
$R^w$ is selected from the group consisting of hydrogen atom, alkyl, $-C(O)R^6$, $-S(O)_2OH$, $-S(O)_2O^-Q^+$, $-PO(OH)_2$, $-PO(OH)O^-Q^+$ and $-PO(O^-)_2W^{2+}$; $Q^+$ is a pharmaceutically acceptable monovalent cation; $W^{2+}$ is a pharmaceutically acceptable divalent cation;
$R^6$ is selected from the group consisting of alkyl, alkoxy, alkenyl, carboxy and carboxylate, wherein the alkyl, alkoxy and alkenyl are each optionally substituted by one or more substituents selected from the group consisting of hydroxy, amino, carboxy and carboxylate;
n is 0, 1, 2, 3 or 4;
s is 0, 1, 2, 3 or 4; and
t is 0, 1 or 2.

In some embodiments, the present disclosure provides a compound of formula (I) or a tautomer, mesomer, racemate, enantiomer, diastereomer thereof, or mixture thereof, or a pharmaceutically acceptable salt thereof,

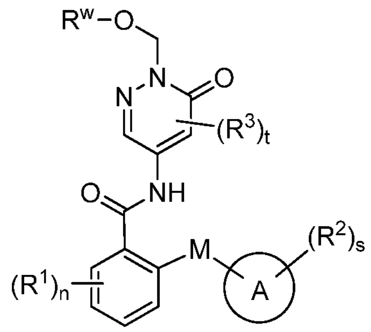

( I )

wherein:

M is selected from the group consisting of O, $CR^4R^5$ and S;
ring A is an aryl or heteroaryl;
each $R^1$ is identical or different and each is independently selected from the group consisting of hydrogen atom, halogen, alkyl, alkoxy, haloalkyl, haloalkoxy, cyano, amino, nitro, hydroxy, hydroxyalkyl, cycloalkyl, heterocyclyl, aryl and heteroaryl;
each $R^2$ is identical or different and each is independently selected from the group consisting of hydrogen atom, halogen, alkyl, alkoxy, haloalkyl, haloalkoxy, cyano, amino, nitro, hydroxy, hydroxyalkyl, cycloalkyl, heterocyclyl, aryl and heteroaryl, wherein the alkyl, cycloalkyl, heterocyclyl, aryl and heteroaryl are each optionally substituted by one or more substituents selected from the group consisting of alkyl, haloalkyl, halogen, amino, nitro, cyano, hydroxy, alkoxy, haloalkoxy, hydroxyalkyl, cycloalkyl, heterocyclyl, aryl and heteroaryl;
each $R^3$ is identical or different and each is independently selected from the group consisting of hydrogen atom, halogen, alkyl, alkoxy, haloalkyl, cyano, amino, nitro, hydroxy, hydroxyalkyl, cycloalkyl, heterocyclyl, aryl and heteroaryl;
$R^4$ and $R^5$ are identical or different and are each independently selected from the group consisting of hydrogen atom, halogen, alkyl, alkoxy, haloalkyl, cyano, amino, nitro, hydroxy, hydroxyalkyl, cycloalkyl, heterocyclyl, aryl and heteroaryl;
$R^w$ is selected from the group consisting of hydrogen atom, alkyl and $-C(O)R^6$;
$R^6$ is selected from the group consisting of alkyl, alkoxy, alkenyl and carboxy, wherein the alkyl, alkoxy and alkenyl are each optionally substituted by one or more substituents selected from the group consisting of hydroxy, amino and carboxy;
n is 0, 1, 2, 3 or 4;
s is 0, 1, 2, 3 or 4; and
t is 0, 1 or 2.

In some embodiments of the present disclosure, in the compound of formula (I) or the tautomer, mesomer, racemate, enantiomer, diastereomer thereof, or mixture thereof, or the pharmaceutically acceptable salt thereof, ring A is a phenyl or pyridyl.

In some embodiments of the present disclosure, in the compound of formula (I) or the tautomer, mesomer, racemate, enantiomer, diastereomer thereof, or mixture thereof, or the pharmaceutically acceptable salt thereof, $R^w$ is selected from the group consisting of hydrogen atom, -C(O)-alkyl, -C(O)-alkoxy, -C(O)-alkylene-COOH, -C(O)-alkenylene-COOH, -C(O)-COOH, $-S(O)_2OH$ and $-C(O)\text{-alkylene-}NH_2$, wherein the alkyl, alkoxy, alkylene and alkenylene are each optionally substituted by one or more hydroxys; and preferably, $R^w$ is selected from the group consisting of hydrogen atom, $-C(O)-C_{1-6}$ alkyl, $-C(O)-C_{1-6}$ alkoxy, $-C(O)-C_{1-6}$ alkylene-COOH, $-C(O)-C_{2-6}$ alkenylene-COOH, -C(O)-COOH, $-S(O)_2OH$ and $-C(O)-C_{1-6}$ alkylene-$NH_2$, wherein the $C_{1-6}$ alkyl, $C_{1-6}$ alkoxy, $C_{1-6}$ alkylene and $C_{2-6}$ alkenylene are each optionally substituted by one or more hydroxys.

In some embodiments of the present disclosure, in the compound of formula (I) or the tautomer, mesomer, racemate, enantiomer, diastereomer thereof, or mixture thereof, or the pharmaceutically acceptable salt thereof, $R^w$ is selected from the group consisting of hydrogen atom, -C(O)-alkyl, -C(O)-alkoxy, -C(O)-alkylene-COOH, -C(O)-alkenylene-COOH, -C(O)-COOH and $-C(O)\text{-alkylene-}NH_2$, wherein the alkyl, alkoxy, alkylene and alkenylene are each optionally substituted by one or more hydroxys.

In some embodiments of the present disclosure, in the compound of formula (I) or the tautomer, mesomer, racemate, enantiomer, diastereomer thereof, or mixture thereof, or the pharmaceutically acceptable salt thereof, $R^w$ is selected from the group consisting of hydrogen atom, $-C(O)CH_3$, $-C(O)CH(OH)CH_3$, $-C(O)CH(CH_3)_2$, $-C(O)OCH_2CH_3$, $-C(O)CH_2COOH$, $-C(O)CH_2CH_2COOH$, $-C(O)CH(OH)CH_2COOH$, $-C(O)CH_2CH(OH)COOH$, -C(O)CH(OH)CH(OH)COOH, -C(O)-CH=CH-COOH, -C(O)-COOH, $-S(O)_2OH$ and $-C(O)CH_2NH_2$, and preferably selected from the group consisting of $-C(O)CH_2COOH$, $-C(O)CH_2CH_2COOH$, $-C(O)CH(OH)CH_2COOH$, $-C(O)CH_2CH(OH)COOH$, -C(O)CH(OH)CH(OH)COOH, -C(O)-CH=CH-COOH and -C(O)-COOH.

In some embodiments of the present disclosure, in the compound of formula (I) or the tautomer, mesomer, racemate, enantiomer, diastereomer thereof, or mixture thereof, or the pharmaceutically acceptable salt thereof, $R^w$ is selected from the group consisting of hydrogen atom, $-C(O)CH_3$, $-C(O)CH(OH)CH_3$, $-C(O)CH(CH_3)_2$, $-C(O)OCH_2CH_3$, $-C(O)CH_2COOH$, $-C(O)CH_2CH_2COOH$, $-C(O)CH(OH)CH_2COOH$, $-C(O)CH_2CH(OH)COOH$, -C(O)CH(OH)CH(OH)COOH, -C(O)-CH=CH-COOH, -C(O)-COOH and $-C(O)CH_2NH_2$, and preferably selected from the group consisting of $-C(O)CH_2COOH$, $-C(O)CH_2CH_2COOH$, $-C(O)CH(OH)CH_2COOH$, $-C(O)CH_2CH(OH)COOH$, -C(O)CH(OH)CH(OH)COOH, -C(O)-CH=CH-COOH and -C(O)-COOH.

In some embodiments of the present disclosure, in the compound of formula (I) or the tautomer, mesomer, racemate, enantiomer, diastereomer thereof, or mixture thereof, or the pharmaceutically acceptable salt thereof, M is an O.

In some embodiments of the present disclosure, the compound of formula (I) or the tautomer, mesomer, racemate, enantiomer, diastereomer thereof, or mixture thereof, or the pharmaceutically acceptable salt thereof is a compound of formula (II) or a tautomer, mesomer, racemate, enantiomer, diastereomer thereof, or mixture thereof, or a pharmaceutically acceptable salt thereof,

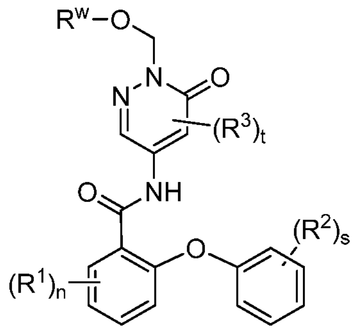

( II )

wherein:
$R^1$, $R^2$, $R^3$, $R^w$, n, s and t are as defined in formula (I).

In some embodiments of the present disclosure, the compound of formula (I) or the tautomer, mesomer, racemate, enantiomer, diastereomer thereof, or mixture thereof, or the pharmaceutically acceptable salt thereof is a compound of formula (IIaa) or a tautomer, mesomer, racemate, enantiomer, diastereomer thereof, or mixture thereof, or a pharmaceutically acceptable salt thereof,

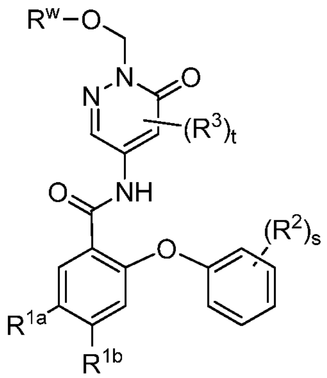

( IIaa )

wherein:

$R^{1a}$ is a halogen; and preferably, $R^{1a}$ is a Cl;
$R^{1b}$ is a haloalkyl; and preferably, $R^{1b}$ is a $CF_3$;
$R^2$, $R^3$, $R^w$, s and t are as defined in formula (I).

In some embodiments of the present disclosure, in the compound of formula (IIaa) or the tautomer, mesomer, racemate, enantiomer, diastereomer thereof, or mixture thereof, or the pharmaceutically acceptable salt thereof, $R^{1a}$ is a chlorine, and $R^{1b}$ is a trifluoromethyl.

In some embodiments of the present disclosure, in the compound of formula (I) or the tautomer, mesomer, racemate, enantiomer, diastereomer thereof, or mixture thereof, or the pharmaceutically acceptable salt thereof, each $R^1$ is identical or different and each is independently selected from the group consisting of hydrogen atom, halogen, alkyl and haloalkyl; and preferably, each $R^1$ is identical or different and each is independently selected from the group consisting of hydrogen atom, halogen, $C_{1-6}$ alkyl and halo$C_{1-6}$ alkyl.

In some embodiments of the present disclosure, in the compound of formula (I) or the tautomer, mesomer, racemate, enantiomer, diastereomer thereof, or mixture thereof, or the pharmaceutically acceptable salt thereof, each $R^2$ is identical or different and each is independently selected from the group consisting of hydrogen atom, halogen,

alkyl, alkoxy, haloalkyl, haloalkoxy, cycloalkyl and cycloalkyloxy; and preferably, each $R^2$ is identical or different and each is independently selected from the group consisting of hydrogen atom, halogen, $C_{1-6}$ alkyl, $C_{1-6}$ alkoxy, halo$C_{1-6}$ alkyl, halo$C_{1-6}$ alkoxy, deuterated $C_{1-6}$ alkoxy and $C_{3-6}$ cycloalkyloxy.

In some embodiments of the present disclosure, in the compound of formula (I) or the tautomer, mesomer, racemate, enantiomer, diastereomer thereof, or mixture thereof, or the pharmaceutically acceptable salt thereof, each $R^2$ is identical or different and each is independently selected from the group consisting of hydrogen atom, halogen, alkyl, alkoxy, deuterated alkoxy, haloalkyl, haloalkoxy, cycloalkyl and cycloalkyloxy; preferably, each $R^2$ is identical or different and each is independently selected from the group consisting of hydrogen atom, halogen, alkyl, alkoxy, haloalkyl, haloalkoxy, deuterated alkoxy and cycloalkyloxy; and more preferably, each $R^2$ is identical or different and each is independently selected from the group consisting of hydrogen atom, halogen, alkyl, alkoxy and deuterated alkoxy.

In some embodiments of the present disclosure, in the compound of formula (I) or the tautomer, mesomer, racemate, enantiomer, diastereomer thereof, or mixture thereof, or the pharmaceutically acceptable salt thereof, n is 0, 1 or 2, and preferably 2.

In some embodiments of the present disclosure, in the compound of formula (I) or the tautomer, mesomer, racemate, enantiomer, diastereomer thereof, or mixture thereof, or the pharmaceutically acceptable salt thereof, $R^3$ is a hydrogen atom.

In some embodiments of the present disclosure, in the compound of formula (I) or the tautomer, mesomer, racemate, enantiomer, diastereomer thereof, or mixture thereof, or the pharmaceutically acceptable salt thereof, s is 0, 1 or 2, and preferably 2.

In some embodiments of the present disclosure, the compound of formula (I) or the tautomer, mesomer, racemate, enantiomer, diastereomer thereof, or mixture thereof, or the pharmaceutically acceptable salt thereof is a compound of formula (III) or a tautomer, mesomer, racemate, enantiomer, diastereomer thereof, or mixture thereof, or a pharmaceutically acceptable salt thereof,

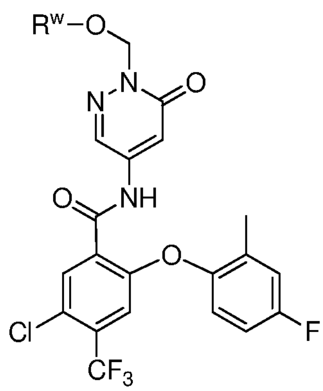

( III )

wherein:
$R^w$ is as defined in formula (I).

In some embodiments of the present disclosure, the compound of formula (I) or the tautomer, mesomer, racemate, enantiomer, diastereomer thereof, or mixture thereof, or the pharmaceutically acceptable salt thereof is a compound of formula (IV) or a tautomer, mesomer, racemate, enantiomer, diastereomer thereof, or mixture thereof, or a pharmaceutically acceptable salt thereof,

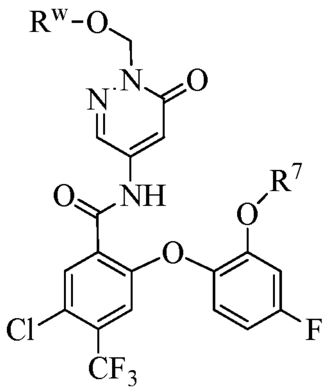

( IV )

wherein:

$R^7$ is selected from the group consisting of alkyl, deuterated alkyl and cycloalkyl; preferably, $R^7$ is selected from the group consisting of $C_{1-6}$ alkyl, deuterated $C_{1-6}$ alkyl and $C_{3-6}$ cycloalkyl; and more preferably, $R^7$ is selected from the group consisting of methyl, deuterated methyl and cyclopropyl; and
$R^w$ is as defined in formula (I).

In some embodiments of the present disclosure, in the compound of formula (IV) or the tautomer, mesomer, racemate, enantiomer, diastereomer thereof, or mixture thereof, or the pharmaceutically acceptable salt thereof, $R^7$ is an alkyl or cycloalkyl, and preferably a methyl or cyclopropyl.

Typical compounds of formula (I) include, but are not limited to:

| Example No. | Structure and name of the compound |
|---|---|
| 1 | 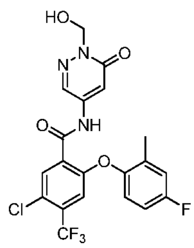 1 |
| | 5-Chloro-2-(4-fluoro-2-methylphenoxy)-*N*-(1-(hydroxymethyl)-6-oxo-1,6 -dihydropyridazin-4-yl)-4-(trifluoromethyl)benzamide 1 |
| 2 | 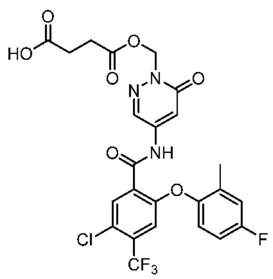 2 |
| | 4-((4-(5-Chloro-2-(4-fluoro-2-methylphenoxy)-4-(trifluoromethyl)benza mido)-6-oxopyridazin-1 (6*H*)-yl)methoxy)-4-oxobutanoic acid **2** |

(continued)

| Example No. | Structure and name of the compound |
|---|---|
| 3 | 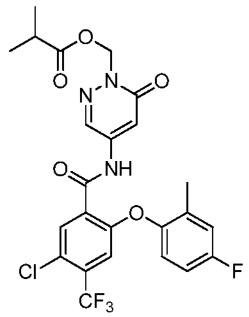 3 |
| | (4-(5-Chloro-2-(4-fluoro-2-methylphenoxy)-4-(trifluoromethyl)benzamid o)-6-oxopyridazin-1 (6*H*)-yl)methyl isobutyrate **3** |
| 4 | 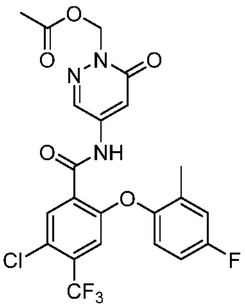 4 |
| | (4-(5-Chloro-2-(4-fluoro-2-methylphenoxy)-4-(trifluoromethyl)benzamido)-6-oxopyridazin-1 (6*H*)-yl)methyl acetate **4** |
| 5 | 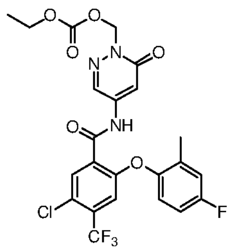 5 |
| | (4-(5-Chloro-2-(4-fluoro-2-methylphenoxy)-4-(trifluoromethyl)benzamid o)-6-oxopyridazin-1 (6*H*)-yl)methyl ethyl carbonate **5** |
| 6d | 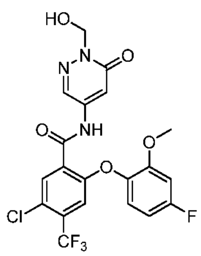 6d |
| | 5-Chloro-2-(4-fluoro-2-methoxyphenoxy)-*N*-(1-(hydroxymethyl)-6-oxo-1 ,6-dihydropyridazin-4-yl)-4-(trifluoromethyl)benzamide **6d** |

(continued)

| Example No. | Structure and name of the compound |
|---|---|
| 6 | 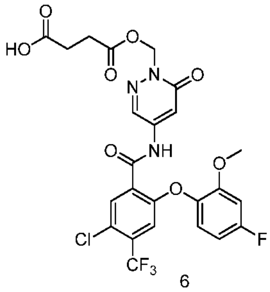 6 |
| | 4-((4-(5-Chloro-2-(4-fluoro-2-methoxyphenoxy)-4-(trifluoromethyl)benza mido)-6-oxopyridazin-1 (6H)-yl)methoxy)-4-oxobutanoic acid 6 |
| 7e | 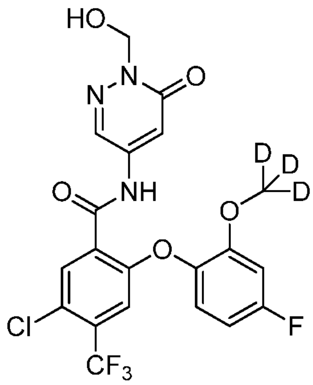 7e |
| | 5-Chloro-2-(4-fluoro-2-(methoxy-$d_3$)phenoxy)-*N*-(1-(hydroxymethyl)-6-o xo-1,6-dihydropyridazin-4-yl)-4-(trifluoromethyl)benzamide **7e** |
| 7 | 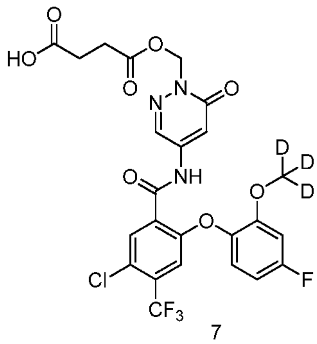 7 |
| | 4-((4-(5-Chloro-2-(4-fluoro-2-(methoxy-$d_3$)phenoxy)-4-(trifluoromethyl)b enzamido)-6-oxopyridazin-1(6*H*)-yl)methoxy)-4-oxobutanoic acid **7** |

(continued)

| Example No. | Structure and name of the compound |
|---|---|
| 8 | 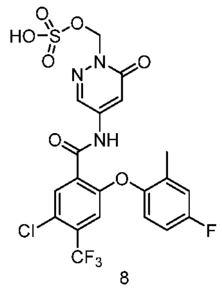 8 |
| | (4-(5-Chloro-2-(4-fluoro-2-methylphenoxy)-4-(trifluoromethyl)benzamid o)-6-oxopyridazin-1(6*H*)-yl)methyl hydrogen sulfate **8** |
| 9 | 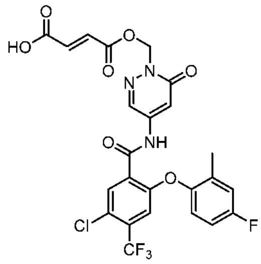 9 |
| | (*E*)-4-((4-(5-Chloro-2-(4-fluoro-2-methylphenoxy)-4-(trifluoromethyl)ben zamido)-6-oxopyridazin-1(6*H*)-yl)methoxy)-4-oxobut-2-enoic acid **9** |
| 10 | 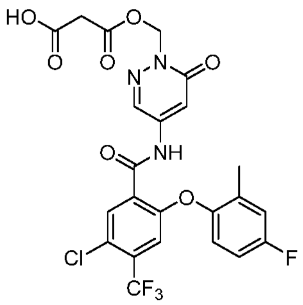 10 |
| | 3-((4-(5-Chloro-2-(4-fluoro-2-methylphenoxy)-4-(trifluoromethyl)benza mido)-6-oxopyridazin-1(6*H*)-yl)methoxy)-3-oxopropanoic acid **10** |
| | 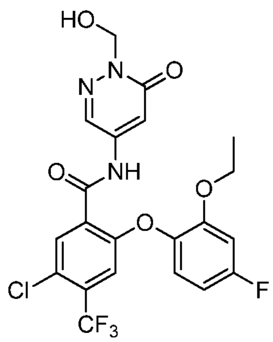 |
| | 5-Chloro-2-(4-fluoro-2-ethoxyphenoxy)-*N*-(1-(hydroxymethyl)-6-oxo-1,6 -dihydropyridazin-4-yl)-4-(trifluoromethyl)benzamide |

(continued)

| Example No. | Structure and name of the compound |
|---|---|
| 11 | 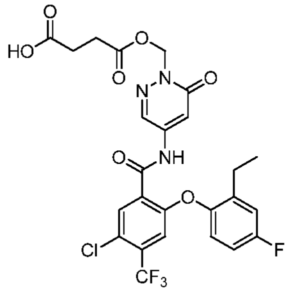 11 |
| | 4-((4-(5-Chloro-2-(2-ethyl-4-fluorophenoxy)-4-(trifluoromethyl)benzami do)-6-oxopyridazin-1(6*H*)-yl)methoxy)-4-oxobutanoic acid **11** |
| 12 | 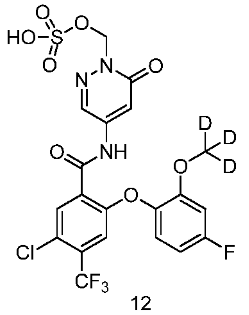 12 |
| | (4-(5-Chloro-2-(4-fluoro-2-(methoxy-$d_3$)phenoxy)-4-(trifluoromethyl)ben zamido)-6-oxopyridazin-1(6*H*)-yl)methyl hydrogen sulfate **12** |
| 13 | 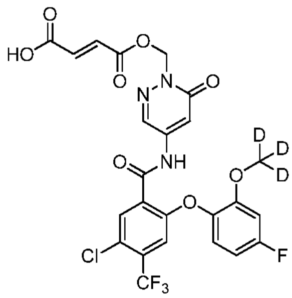 13 |
| | (*E*)-4-((4-(5-Chloro-2-(4-fluoro-2-(methoxy-$d_3$)phenoxy)-4-(trifluorometh yl)benzamido)-6-oxopyridazin-1(6*H*)-yl)methoxy)-4-oxobut-2-enoic acid 13 |
| | 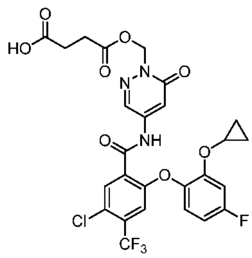 |
| | 4-((4-(5-Chloro-2-(2-cyclopropoxy-4-fluorophenoxy)-4-(trifluoromethyl) benzamido)-6-oxopyridazin-1(6*H*)-yl)methoxy)-4-oxobutanoic acid |

(continued)

| Example No. | Structure and name of the compound |
|---|---|
| | 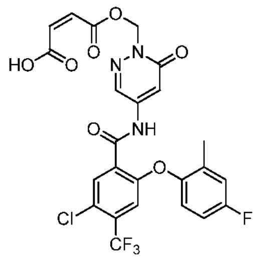 |
| | (Z)-4-((4-(5-Chloro-2-(4-fluoro-2-methylphenoxy)-4-(trifluoromethyl)ben zamido)-6-oxopyridazin-1 (6*H*)-yl)methoxy)-4-oxobut-2-enoic acid |
| | 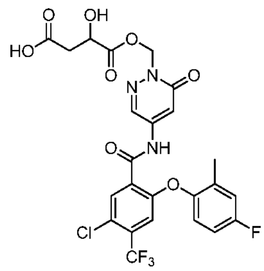 |
| | 4-((4-(5-Chloro-2-(4-fluoro-2-methylphenoxy)-4-(trifluoromethyl)benza mido)-6-oxopyridazin-1 (6*H*)-yl)methoxy)-3-hydroxy-4-oxobutanoic acid |
| | 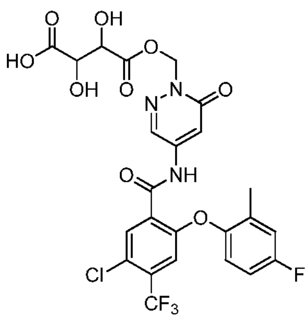 |
| | 4-((4-(5-Chloro-2-(4-fluoro-2-methylphenoxy)-4-(trifluoromethyl)benza mido)-6-oxopyridazin-1 (6*H*)-yl)methoxy)-2,3-dihydroxy-4-oxobutanoic acid |
| | 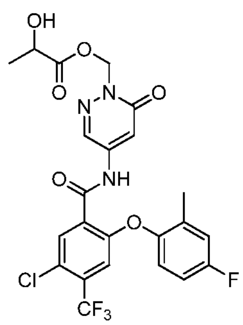 |
| | (4-(5-Chloro-2-(4-fluoro-2-methylphenoxy)-4-(trifluoromethyl)benzamid o)-6-oxopyri dazin-1 (6*H*)-yl)methyl 2-hydroxypropanoate |

(continued)

| Example No. | Structure and name of the compound |
|---|---|
| | 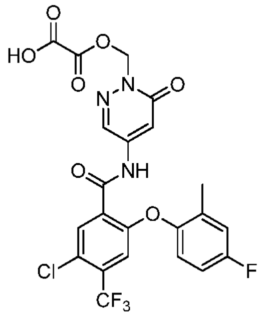 |
| | 2-((4-(5-Chloro-2-(4-fluoro-2-methylphenoxy)-4-(trifluoromethyl)benza mido)-6-oxopyridazin-1(6*H*)-yl)methoxy)-3-oxoacetic acid |
| | 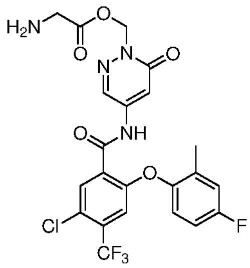 |
| | (4-(5-Chloro-2-(4-fluoro-2-methylphenoxy)-4-(trifluoromethyl)benzamid o)-6-oxopyridazin-1(6*H*)-yl)methyl 2-aminoacetate |
| | 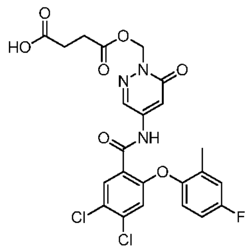 |
| | 4-((4-(4,5-Dichloro-2-(4-fluoro-2-methylphenoxy)benzamido)-6-oxopyrid azin-1(6*H*)-yl)methoxy)-4-oxobutanoic acid |
| | 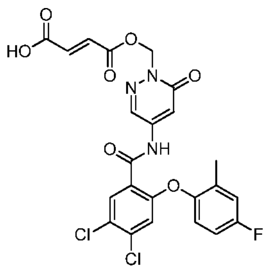 |
| | (*E*)-4-((4-(4,5-Dichloro-2-(4-fluoro-2-methylphenoxy)benzamido)-6-oxop yridazin-1(6*H*)-yl)methoxy)-4-oxobut-2-enoic acid |

(continued)

| Example No. | Structure and name of the compound |
|---|---|
| | 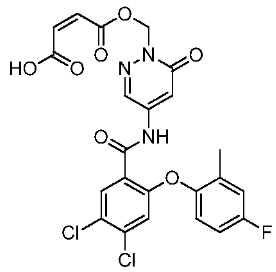 |
| | (Z)-4-((4-(4,5-Dichloro-2-(4-fluoro-2-methylphenoxy)benzamido)-6-oxop yridazin-1(6*H*)-yl) methoxy)-4-oxobut-2-enoic acid |
| | 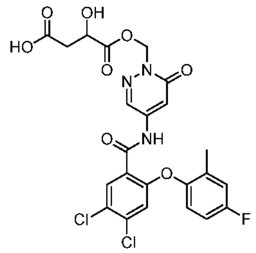 |
| | 4-((4-(4,5-Dichloro-2-(4-fluoro-2-methylphenoxy)benzamido)-6-oxopyrid azin-1(6*H*)-yl)methoxy)-3-hydroxy-4-oxobutanoic acid |
| | 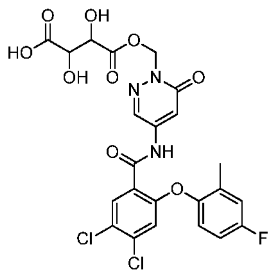 |
| | 4-((4-(4,5-Dichloro-2-(4-fluoro-2-methylphenoxy)benzamido)-6-oxopyrid azin-1(6*H*)-yl)methoxy)-2,3-dihydroxy-4-oxobutanoic acid |
| | 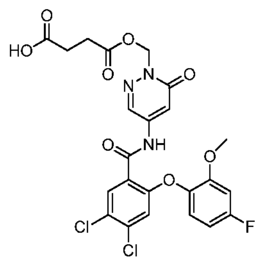 |
| | 4-((4-(4,5-Dichloro-2-(4-fluoro-2-methoxyphenoxy)benzamido)-6-oxopyr idazin-1(6*H*)-yl) methoxy)-4-oxobutanoic acid |

(continued)

| Example No. | Structure and name of the compound |
| --- | --- |
| | 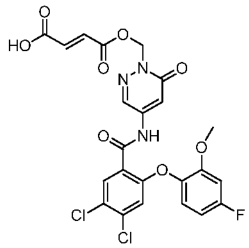 |
| | (*E*)-4-((4-(4,5-Dichloro-2-(4-fluoro-2-methoxyphenoxy)benzamido)-6-ox opyridazin-1(6*H*)-yl) methoxy)-4-oxobut-2-enoic acid |
| | 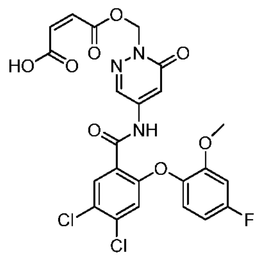 |
| | (*Z*)-4-((4-(4,5-Dichloro-2-(4-fluoro-2-methoxyphenoxy)benzamido)-6-ox opyridazin-1(6*H*)-yl) methoxy)-4-oxobut-2-enoic acid |
| | 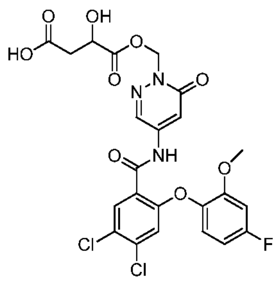 |
| | 4-((4-(4,5-Dichloro-2-(4-fluoro-2-methoxyphenoxy)benzamido)-6-oxopyr idazin-1(6*H*)-yl) methoxy)-3-hydroxy-4-oxobutanoic acid |
| | 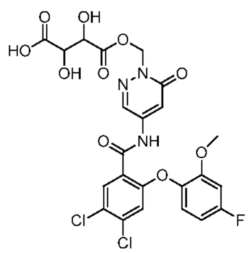 |
| | 4-((4-(4,5-Dichloro-2-(4-fluoro-2-methoxyphenoxy)benzamido)-6-oxopyr idazin-1(6*H*)-yl) methoxy)-2,3-dihydroxy-4-oxobutanoic acid |

or a tautomer, mesomer, racemate, enantiomer, diastereomer thereof, or mixture thereof, or a pharmaceutically acceptable salt thereof.

In another aspect, the present disclosure relates to a method for preparing the compound of formula (I) or the tautomer, mesomer, racemate, enantiomer, diastereomer thereof, or mixture thereof, or the pharmaceutically acceptable salt thereof, comprising a step of:

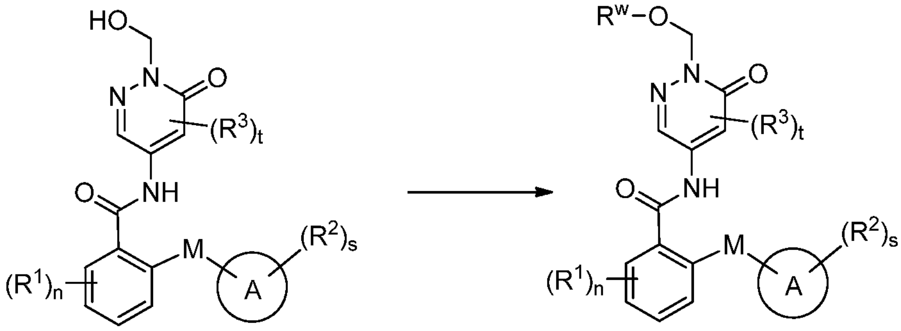

( IA ) ( I )

reacting a compound of formula (IA) with $R^w$-X,

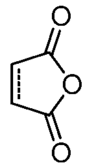

or sulfur trioxide pyridine to obtain the compound of formula (I);
wherein:

$R^w$ is -C(O)$R^6$ or -S(O)$_2$OH;
X is a halogen or hydroxy;
----- is a single bond or double bond; and
ring A, M, $R^1$, $R^2$, $R^3$, $R^6$, n, s and t are as defined in formula (I).

In another aspect, the present disclosure relates to a method for preparing the compound of formula (I) or the tautomer, mesomer, racemate, enantiomer, diastereomer thereof, or mixture thereof, or the pharmaceutically acceptable salt thereof, comprising a step of:

reacting a compound of formula (IA) with $R^w$-X or

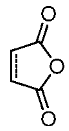

to obtain the compound of formula (I);
wherein: $R^w$ is -C(O)$R^6$; X is a halogen; and $R^6$ is as defined in formula (I).

In a preferred embodiment of the present disclosure, the aforementioned method for preparing the compound of formula (I) or the tautomer, mesomer, racemate, enantiomer, diastereomer thereof, or mixture thereof, or the pharmaceutically acceptable salt thereof, further comprises a step of:

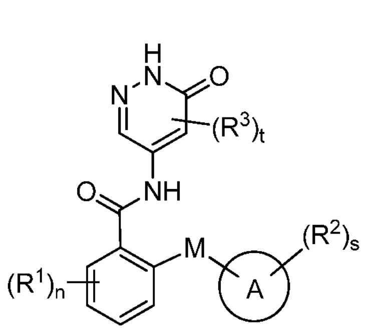

( IB ) ( IA )

reacting a compound of formula (IB) with formaldehyde solution to obtain the compound of formula (IA);
wherein:
ring A, M, $R^1$, $R^2$, $R^3$, n, s and t are as defined in formula (I).

In another aspect, the present disclosure relates to a method for preparing the compound of formula (II) or the tautomer, mesomer, racemate, enantiomer, diastereomer thereof, or mixture thereof, or the pharmaceutically acceptable salt thereof, comprising a step of:

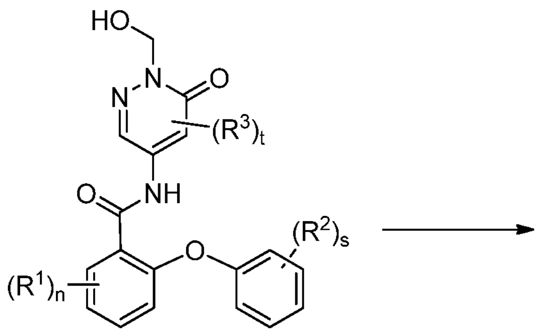

( IIA ) ( II )

reacting a compound of formula (IIA) with $R^w$-X,

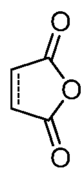

or sulfur trioxide pyridine to obtain the compound of formula (II);
wherein:

$R^w$ is -C(O)$R^6$ or -$S(O)_2OH$;
X is a halogen or hydroxy;
----- is a single bond or double bond;
$R^1$, $R^2$, $R^3$, $R^6$, n, s and t are as defined in formula (II).

In a preferred embodiment of the present disclosure, the aforementioned method for preparing the compound of formula (II) or the tautomer, mesomer, racemate, enantiomer, diastereomer thereof, or mixture thereof, or the pharmaceutically acceptable salt thereof, further comprises a step of:

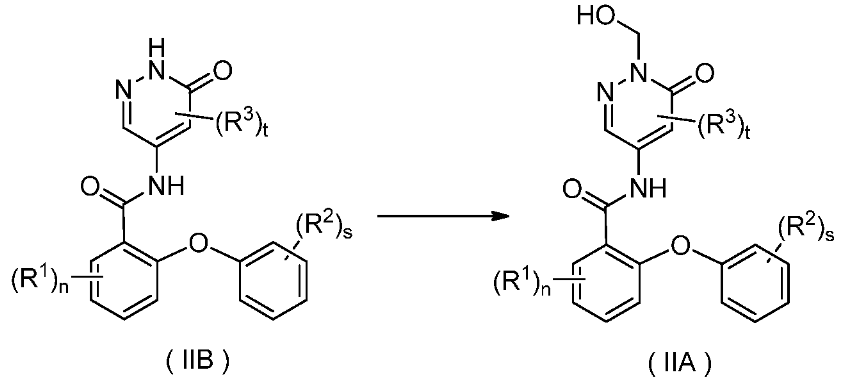

( IIB ) ( IIA )

reacting a compound of formula (IIB) with formaldehyde solution to obtain the compound of formula (IIA);
wherein:
$R^1$, $R^2$, $R^3$, n, s and t are as defined in formula (II).

In another aspect, the present disclosure relates to a method for preparing the compound of formula (IIaa) or the tautomer, mesomer, racemate, enantiomer, diastereomer thereof, or mixture thereof, or the pharmaceutically acceptable salt thereof, comprising a step of:

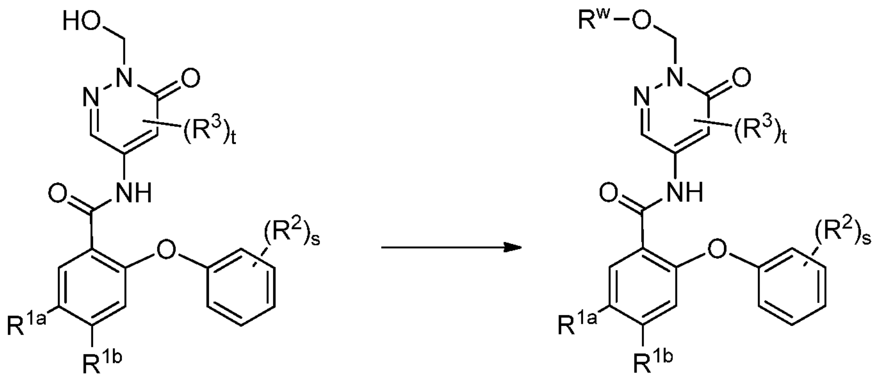

( IIaa-A ) ( IIaa )

reacting a compound of formula (IIaa-A) with $R^w$-X,

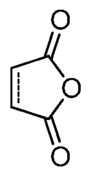

or sulfur trioxide pyridine to obtain the compound of formula (IIaa);
wherein:

$R^w$ is -C(O)$R^6$ or -$S(O)_2OH$;
X is a halogen or hydroxy;
===== is a single bond or double bond; and
$R^{1a}$, $R^{1b}$, $R^2$, $R^3$, $R^6$, s and t are as defined in formula (IIaa).

In a preferred embodiment of the present disclosure, the aforementioned method for preparing the compound of formula (IIaa) or the tautomer, mesomer, racemate, enantiomer, diastereomer thereof, or mixture thereof, or the pharmaceutically acceptable salt thereof, further comprises a step of:

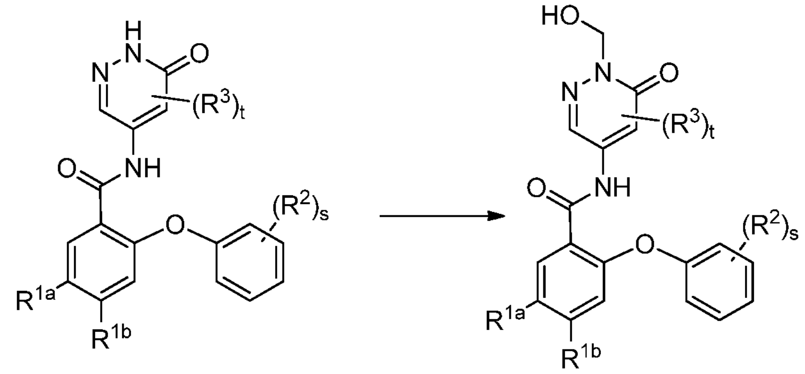

( IIaa-B ) ( IIaa-A )

reacting a compound of formula (IIaa-B) with formaldehyde solution to obtain the compound of formula (IIaa-A);
wherein:
$R^{1a}$, $R^{1b}$, $R^2$, $R^3$, s and t are as defined in formula (IIaa).

In another aspect, the present disclosure relates to a method for preparing the compound of formula (III) or the tautomer, mesomer, racemate, enantiomer, diastereomer thereof, or mixture thereof, or the pharmaceutically acceptable salt thereof, comprising a step of:

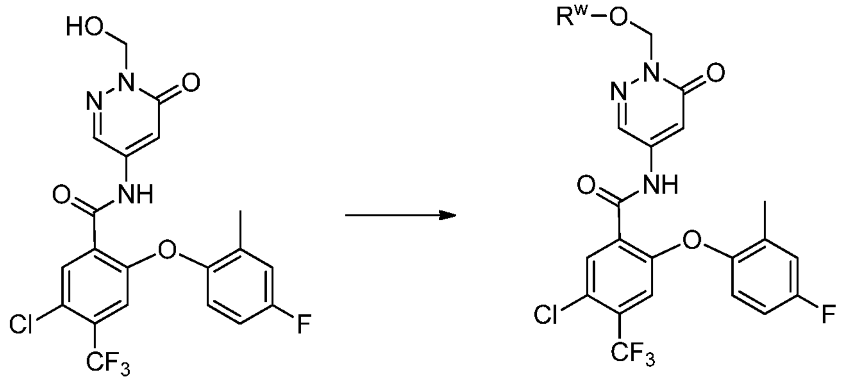

1 ( III )

reacting compound 1 with $R^w$-X,

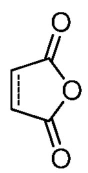

or sulfur trioxide pyridine to obtain the compound of formula (III);
wherein:

$R^w$ is -C(O)$R^6$ or -S(O)$_2$OH;
X is a halogen or hydroxy;
===== is a single bond or double bond; and
$R^6$ is as defined in formula (III).

In a preferred embodiment of the present disclosure, the aforementioned method for preparing the compound of formula (III) or the tautomer, mesomer, racemate, enantiomer, diastereomer thereof, or mixture thereof, or the pharmaceutically acceptable salt, thereof further comprises a step of:

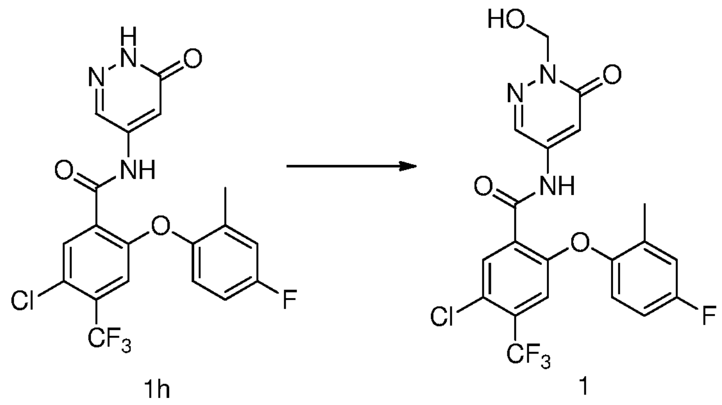

reacting compound 1h with formaldehyde solution to obtain compound 1.

In another aspect, the present disclosure relates to a method for preparing the compound of formula (IV) or the tautomer, mesomer, racemate, enantiomer, diastereomer thereof, or mixture thereof, or the pharmaceutically acceptable salt thereof, comprising a step of:

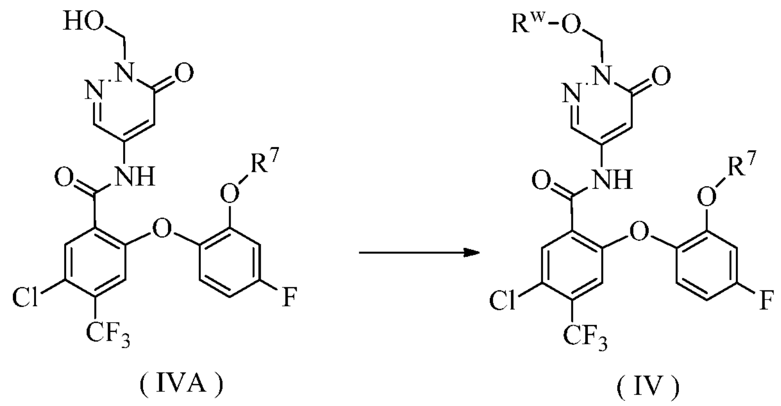

reacting a compound of formula (IVA) with $R^w$-X,

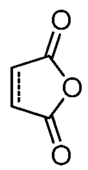

or sulfur trioxide pyridine to obtain the compound of formula (IV);
wherein:

$R^w$ is $-C(O)R^6$ or $-S(O)_2OH$;
X is a halogen or hydroxy;
===== is a single bond or double bond; and
$R^6$ and $R^7$ are as defined in formula (IV).

In a preferred embodiment of the present disclosure, the aforementioned method for preparing the compound of formula (IVA) or the tautomer, mesomer, racemate, enantiomer, diastereomer thereof, or mixture thereof, or the pharmaceutically acceptable salt thereof, further comprises a step of:

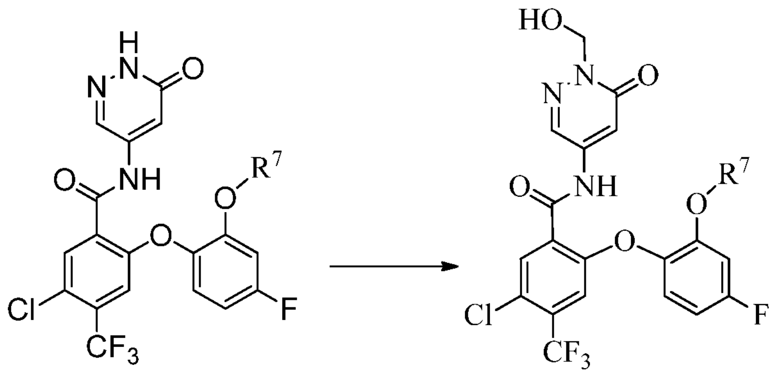

( IVB ) ( IVA )

reacting a compound of formula (IVB) with formaldehyde solution to obtain the compound of formula (IVA); wherein: $R^7$ is as defined in formula (IV).

In another aspect, the present disclosure relates to a pharmaceutical composition comprising the aforementioned compound of formula (I) or the tautomer, mesomer, racemate, enantiomer, diastereomer thereof, or mixture thereof, or the pharmaceutically acceptable salt thereof, and one or more pharmaceutically acceptable carriers, diluents or excipients.

The present disclosure also relates to a method for preparing the aforementioned pharmaceutical composition, comprising a step of mixing the aforementioned compound of formula (I), or the tautomer, mesomer, racemate, enantiomer, diastereomer thereof, or mixture thereof, or the pharmaceutically acceptable salt thereof with the pharmaceutically acceptable carriers, diluents or excipients.

The present disclosure also relates to a use of the aforementioned compound of formula (I), or the tautomer, mesomer, racemate, enantiomer, diastereomer thereof, or mixture thereof, or the pharmaceutically acceptable salt thereof, or the aforementioned pharmaceutical composition in the preparation of a medicament for inhibiting the voltage-gated sodium channel in a subject. The voltage-gated sodium channel is preferaby $Na_V1.8$.

The present disclosure also relates to a use of the aforementioned compound of formula (I), or the tautomer, mesomer, racemate, enantiomer, diastereomer thereof, or mixture thereof, or the pharmaceutically acceptable salt thereof, or the aforementioned pharmaceutical composition in the preparation of a medicament for treating and/or alleviating pain and pain-related diseases, multiple sclerosis, Charcot-Marie-Tooth syndrome, incontinence or cardiac arrhythmia. The pain is preferaby selected from the group consisting of chronic pain, acute pain, inflammatory pain, cancer pain, neuropathic pain, musculoskeletal pain, primary pain, intestinal pain and idiopathic pain.

The present disclosure also relates to a method for inhibiting the voltage-gated sodium channel in a subject, comprising a step of administering to the subject in need thereof the aforementioned compound of formula (I), or the tautomer, mesomer, racemate, enantiomer, diastereomer thereof, or mixture thereof, or the pharmaceutically acceptable salt thereof, or the aforementioned pharmaceutical composition. The voltage-gated sodium channel is preferaby $Na_V1.8$.

The present disclosure also relates to a method for treating and/or alleviating pain and pain-related diseases, multiple sclerosis, Charcot-Marie-Tooth syndrome, incontinence or cardiac arrhythmia, comprising a step of administering to a patient in need thereof the aforementioned compound of formula (I), or the tautomer, mesomer, racemate, enantiomer, diastereomer thereof, or mixture thereof, or the pharmaceutically acceptable salt thereof, or the aforementioned pharmaceutical composition. The pain is preferaby selected from the group consisting of chronic pain, acute pain, inflammatory pain, cancer pain, neuropathic pain, musculoskeletal pain, primary pain, intestinal pain and idiopathic pain.

The present disclosure also relates to the compound of formula (I), or the tautomer, mesomer, racemate, enantiomer, diastereomer thereof, or mixture thereof, or the pharmaceutically acceptable salt thereof, or the aforementioned pharmaceutical composition, for use as a medicament.

The present disclosure also relates to the compound of formula (I), or the tautomer, mesomer, racemate, enantiomer, diastereomer thereof, or mixture thereof, or the pharmaceutically acceptable salt thereof, or the aforementioned pharmaceutical composition, for use in inhibiting the voltage-gated sodium channel in a subject. The voltage-gated sodium channel is preferaby $Na_V1.8$.

The present disclosure also relates to the aforementioned compound of formula (I), or the tautomer, mesomer, racemate, enantiomer, diastereomer thereof, or mixture thereof, or the pharmaceutically acceptable salt thereof, or the aforementioned pharmaceutical composition, for use in treating and/or alleviating pain and pain-related diseases, multiple sclerosis, Charcot-Marie-Tooth syndrome, incontinence or cardiac arrhythmia. The pain is preferaby selected from the group consisting of chronic pain, acute pain, inflammatory pain, cancer pain, neuropathic pain, musculoskeletal pain,

primary pain, intestinal pain and idiopathic pain.

The neuropathic pain in the present disclosure is preferaby selected from the group consisting of trigeminal neuralgia, postherpetic neuralgia, diabetic neuralgia, painful HIV-associated sensory neuropathy, burning syndrome, post-amputation pain, post spinal cord injury pain, phantom pain, painful neuroma, traumatic neuroma, Morton neuroma, nerve crush injury, spinal canal stenosis, carpal tunnel syndrome, radicular pain, sciatica pain, nerve avulsion, brachial plexus avulsion injury, complex regional pain syndrome, neuralgia caused by drug therapy, neuralgia caused by cancer chemotherapy, neuralgia caused by antiretroviral therapy, primary small fiber neuropathy, primary sensory neuralgia and trigeminal autonomic headache.

The musculoskeletal pain in the present disclosure is preferaby selected from the group consisting of osteoarthritis pain, back pain, cold pain, burning pain and dental pain.

The intestinal pain in the present disclosure is preferaby selected from the group consisting of inflammatory bowel disease pain, Crohn's disease pain and interstitial cystitis pain.

The inflammatory pain in the present disclosure is preferaby selected from the group consisting of rheumatoid arthritis pain and vulvar pain.

The idiopathic pain in the present disclosure is preferaby fibromyalgia.

The dosage of the compound or composition used in the treatment method of the present disclosure will generally vary according to the severity of the disease, the weight of the patient, and the relative efficacy of the compound. However, as a general guide, a suitable unit dose can be 0.1 to 1000 mg.

In addition to the active compound, the pharmaceutical composition of the present disclosure can also comprise one or more auxiliaries including a filler (diluent), binder, wetting agent, disintegrant, excipient and the like. Depending on the administration mode, the composition can comprise 0.1 to 99% by weight of the active compound.

The pharmaceutical composition containing the active ingredient can be in a form suitable for oral administration, for example, a tablet, troche, lozenge, aqueous or oily suspension, dispersible powder or granule, emulsion, hard or soft capsule, syrup or elixir. An oral composition can be prepared according to any known method in the art for the preparation of pharmaceutical composition. Such a composition can contain one or more ingredient(s) selected from the group consisting of sweeteners, flavoring agents, colorants and preservatives, in order to provide a pleasing and palatable pharmaceutical formulation. The tablet contains the active ingredient in admixture with nontoxic, pharmaceutically acceptable excipients suitable for the manufacture of tablets. These excipients can be inert excipients, granulating agents, disintegrating agents and lubricants. The tablet can be uncoated or coated by means of a known technique to mask drug taste or delay the disintegration and absorption of the active ingredient in the gastrointestinal tract, thereby providing sustained release over a long period of time.

An oral formulation can also be provided as soft gelatin capsules in which the active ingredient is mixed with an inert solid diluent, or the active ingredient is mixed with a water-soluble carrier, an oil medium or olive oil.

An aqueous suspension comprises an active ingredient in admixture with excipients suitable for the manufacture of an aqueous suspension. Such excipients are suspending agents, dispersants or wetting agents. The aqueous suspension can also comprise one or more preservatives such as ethyl paraben or *n*-propyl paraben, one or more colorants, one or more flavoring agents, and one or more sweeteners.

An oil suspension can be formulated by suspending the active ingredient in a vegetable oil or mineral oil. The oil suspension can contain a thickener. The aforementioned sweeteners and flavoring agents can be added to provide a palatable formulation. These compositions can be preserved by adding an antioxidant.

The active ingredient in admixture with the dispersants or wetting agents, suspending agents or one or more preservatives can be prepared as dispersible powders or granules suitable for the preparation of an aqueous suspension by adding water. Suitable dispersants or wetting agents and suspending agents are exemplified by those already mentioned above. Additional excipients, such as sweeteners, flavoring agents and colorants, can also be added.

The pharmaceutical composition of the present disclosure can also be in the form of an oil-in-water emulsion. The oil phase can be a vegetable oil, or a mineral oil (such as liquid paraffin), or a mixture thereof. Suitable emulsifying agents can be naturally occurring phospholipids or partial esters. The emulsion can also contain a sweetening agent, flavoring agent, preservative and antioxidant.

The pharmaceutical composition of the present disclosure can be in the form of a sterile injectable aqueous solution. Acceptable vehicles or solvents that can be used are water, Ringer's solution or isotonic sodium chloride solution. The sterile injectable formulation can be a sterile injectable oil-in-water micro-emulsion in which the active ingredient is dissolved in the oil phase. The injectable solution or micro-emulsion can be introduced into a patient's bloodstream by local bolus injection.

The pharmaceutical composition of the present disclosure can be in the form of a sterile injectable aqueous or oily suspension for intramuscular and subcutaneous administration. Such a suspension can be formulated with suitable dispersants or wetting agents and suspending agents as described above according to known techniques. The sterile injectable formulation can also be a sterile injectable solution or suspension prepared in a nontoxic parenterally acceptable diluent or solvent. Moreover, sterile fixed oils can easily be used as a solvent or suspending medium.

The compound of the present disclosure can be administered in the form of a suppository for rectal administration. These pharmaceutical compositions can be prepared by mixing the drug with a suitable non-irritating excipient that is solid at ordinary temperatures, but liquid in the rectum, thereby melting in the rectum to release the drug.

It is well known to those skilled in the art that the dosage of a drug depends on a variety of factors including but not limited to, the following factors: activity of a specific compound, age of the patient, weight of the patient, general health of the patient, behavior of the patient, diet of the patient, administration time, administration route, excretion rate, drug combination and the like. In addition, the optimal treatment, such as treatment mode, daily dose of the compound of formula (I) or the type of pharmaceutically acceptable salt thereof can be verified by traditional therapeutic regimens.

## TERM DEDINITIONS

Unless otherwise stated, the terms used in the specification and claims have the meanings described below.

The term "alkyl" refers to a saturated aliphatic hydrocarbon group, which is a straight or branched chain group comprising 1 to 20 carbon atoms, preferably an alkyl having 1 to 12 carbon atoms, and more preferably an alkyl having 1 to 6 carbon atoms. Non-limiting examples include methyl, ethyl, *n*-propyl, isopropyl, *n*-butyl, isobutyl, *tert-butyl, sec*-butyl, *n*-pentyl, 1,1-dimethylpropyl, 1,2-dimethylpropyl, 2,2-dimethylpropyl, 1-ethylpropyl, 2-methylbutyl, 3-methylbutyl, *n*-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, *n*-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, *n-octyl,* 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, *n*-nonyl, 2-methyl-2-ethylhexyl, 2-methyl-3-ethylhexyl, 2,2-diethylpentyl, n-decyl, 3,3-diethylhexyl, 2,2-diethylhexyl, and various branched isomers thereof. More preferably, the alkyl group is a lower alkyl having 1 to 6 carbon atoms, and non-limiting examples include methyl, ethyl, *n*-propyl, isopropyl, *n*-butyl, isobutyl, *tert-butyl, sec*-butyl, *n*-pentyl, 1,1-dimethylpropyl, 1,2-dimethylpropyl, 2,2-dimethylpropyl, 1-ethylpropyl, 2-methylbutyl, 3-methylbutyl, *n*-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl and the like. The alkyl can be substituted or unsubstituted. When substituted, the substituent group(s) can be substituted at any available connection point. The substituent group(s) is preferably one or more groups independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, oxo, carboxy and alkoxycarbonyl.

The term "alkylene" refers to a saturated linear or branched aliphatic hydrocarbon group having two residues derived from the removal of two hydrogen atoms from the same carbon atom or two different carbon atoms of the parent alkane. It is a linear or branched alkylene having 1 to 20 carbon atoms, preferably 1 to 12 carbon atoms, and more preferably 1 to 6 carbon atoms. Non-limiting examples of alkylene include, but are not limited to, methylene ($-CH_2-$), 1,1-ethylene ($-CH(CH_3)-$), 1,2-ethylene ($-CH_2CH_2)-$, 1,1-propylene ($-CH(CH_2CH_3)-$), 1,2-propylene ($-CH_2CH(CH_3)-$), 1,3-propylene ($-CH_2CH_2CH_2-$), 1,4-butylene ($-CH_2CH_2CH_2CH_2-$) and the like. The alkylene can be substituted or unsubstituted. When substituted, the substituent group(s) can be substituted at any available connection point. The substituent group(s) is preferably one or more groups independently optionally selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio and oxo.

The term "alkenyl" refers to an alkyl containing carbon-carbon double bond(s) in the molecule, wherein the definition of the alkyl is as described above. The alkenyl can be substituted or unsubstituted. When substituted, the substituent group(s) is preferably one or more groups independently selected from the group consisting of hydrogen atom, alkyl, alkoxy, halogen, haloalkyl, hydroxy, hydroxyalkyl, cyano, amino, nitro, cycloalkyl, heterocyclyl, aryl and heteroaryl.

The term "alkenylene" refers to an alkenyl as described above having two residues derived from the removal of two hydrogen atoms from two different carbon atoms of the parent alkenyl. The alkenylene can be substituted or unsubstituted. When substituted, the substituent group(s) is preferably one or more groups independently selected from the group consisting of hydrogen atom, alkyl, alkoxy, halogen, haloalkyl, hydroxy, hydroxyalkyl, cyano, amino, nitro, cycloalkyl, heterocyclyl, aryl and heteroaryl.

The term "alkoxy" refers to an -O-(alkyl) or an -O-(unsubstituted cycloalkyl) group, wherein the alkyl is as defined above. Non-limiting examples of alkoxy include methoxy, ethoxy, propoxy, butoxy, cyclopropyloxy, cyclobutyloxy, cyclopentyloxy, cyclohexyloxy. The alkoxy can be optionally substituted or unsubstituted. When substituted, the substituent group(s) is preferably one or more groups independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, carboxy and alkoxycarbonyl.

The term "cycloalkyl" refers to a saturated or partially unsaturated monocyclic or polycyclic hydrocarbon substituent group having 3 to 20 carbon atoms, preferably 3 to 12 carbon atoms, more preferably 3 to 6 carbon atoms (for example 3, 4, 5 or 6 carbon atoms), and most preferably 5 to 6 carbon atoms. Non-limiting examples of monocyclic cycloalkyl include cyclopropyl, cyclobutyl, cyclopentyl, cyclopentenyl, cyclohexyl, cyclohexenyl, cyclohexadienyl, cycloheptyl, cycloheptatrienyl, cyclooctyl and the like. Polycyclic cycloalkyl includes a cycloalkyl having a spiro ring, fused ring or bridged ring.

The term "spiro cycloalkyl" refers to a 5 to 20 membered polycyclic group with individual rings connected through one shared carbon atom (called a spiro atom), wherein the rings can contain one or more double bonds, but none of the rings has a completely conjugated π-electron system. The spiro cycloalkyl is preferably a 6 to 14 membered spiro cycloalkyl, and more preferably a 7 to 10 membered spiro cycloalkyl (for example 7, 8, 9 or 10 membered spiro cycloalkyl). According to the number of the spiro atoms shared between the rings, the spiro cycloalkyl can be divided into a mono-spiro cycloalkyl, di-spiro cycloalkyl, or poly-spiro cycloalkyl, and the spiro cycloalkyl is preferably a mono-spiro cycloalkyl or di-spiro cycloalkyl, and more preferably a 4-membered/4-membered, 4-membered/5-membered, 4-membered/6-membered, 5-membered/5-membered, or 5-membered/6-membered mono-spiro cycloalkyl. Non-limiting examples of spiro cycloalkyl include:

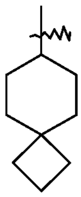 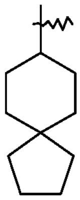 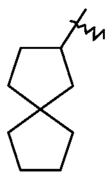 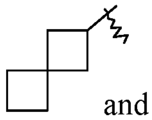 and 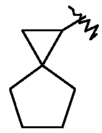 .

The term "fused cycloalkyl" refers to a 5 to 20 membered all-carbon polycyclic group, wherein each ring in the system shares an adjacent pair of carbon atoms with another ring, one or more rings can contain one or more double bonds, but none of the rings has a completely conjugated π-electron system. The fused cycloalkyl is preferably a 6 to 14 membered fused cycloalkyl, and more preferably a 7 to 10 membered fused cycloalkyl. According to the number of membered rings, the fused cycloalkyl can be divided into a bicyclic, tricyclic, tetracyclic or polycyclic fused cycloalkyl, and the fused cycloalkyl is preferably a bicyclic or tricyclic fused cycloalkyl, and more preferably a 5-membered/5-membered, or 5-membered/6-membered bicyclic fused cycloalkyl. Non-limiting examples of fused cycloalkyl include:

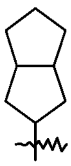 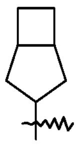 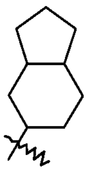 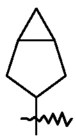 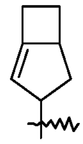 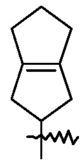 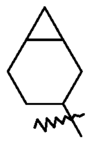 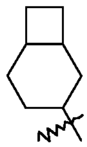 and 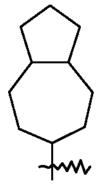

The term "bridged cycloalkyl" refers to a 5 to 20 membered all-carbon polycyclic group, wherein every two rings in the system share two disconnected carbon atoms, the rings can have one or more double bonds, but none of the rings has a completely conjugated π-electron system. The bridged cycloalkyl is preferably a 6 to 14 membered bridged cycloalkyl, and more preferably a 7 to 10 membered bridged cycloalkyl. According to the number of membered rings, the bridged cycloalkyl can be divided into a bicyclic, tricyclic, tetracyclic or polycyclic bridged cycloalkyl, and the bridged cycloalkyl is preferably a bicyclic, tricyclic or tetracyclic bridged cycloalkyl, and more preferably a bicyclic or tricyclic bridged cycloalkyl. Non-limiting examples of bridged cycloalkyl include:

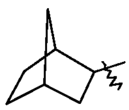 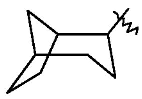 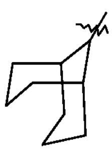 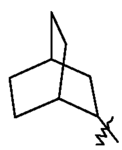 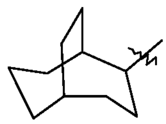

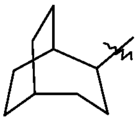 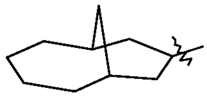 and 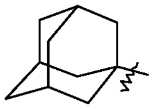.

The cycloalkyl (including cycloalkyl, spiro cycloalkyl, fused cycloalkyl and bridged cycloalkyl) ring can be fused to the ring of aryl, heteroaryl or heterocyclyl, wherein the ring bound to the parent structure is cycloalkyl. Non-limiting examples include indanyl, tetrahydronaphthyl, benzocycloheptyl and the like, and preferably benzocyclopentyl, tetrahydronaphthyl. The cycloalkyl can be optionally substituted or unsubstituted. When substituted, the substituent group(s) is preferably one or more groups independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, oxo, carboxy and alkoxycarbonyl.

The term "heterocyclyl" refers to a 3 to 20 membered saturated or partially unsaturated monocyclic or polycyclic hydrocarbon substituent group, wherein one or more ring atoms are heteroatoms selected from the group consisting of N, O and $S(O)_m$ (wherein m is an integer of 0 to 2), but excluding -O-O-, -O-S- or -S-S- in the ring, with the remaining ring atoms being carbon atoms. Preferably, the heterocyclyl has 3 to 12 ring atoms wherein 1 to 4 atoms are heteroatoms; most preferably, 3 to 8 ring atoms wherein 1 to 3 atoms are heteroatoms; and most preferably 5 to 6 ring atoms wherein 1 to 2 or 1 to 3 atoms are heteroatoms. Non-limiting examples of monocyclic heterocyclyl include pyrrolidinyl, imidazolidinyl, tetrahydrofuranyl, tetrahydropyranyl, tetrahydrothienyl, dihydroimidazolyl, dihydrofuranyl, dihydropyrazolyl, dihydropyrrolyl, piperidinyl, piperazinyl, morpholinyl, thiomorpholinyl, homopiperazinyl and the like, and preferably tetrahydropyranyl, piperidinyl, pyrrolidinyl. Polycyclic heterocyclyl includes a heterocyclyl having a spiro ring, fused ring or bridged ring.

The term "spiro heterocyclyl" refers to a 5 to 20 membered polycyclic heterocyclyl group with individual rings connected through one shared atom (called a spiro atom), wherein one or more ring atoms are heteroatoms selected from the group consisting of N, O and $S(O)_m$ (wherein m is an integer of 0 to 2), with the remaining ring atoms being carbon atoms, where the rings can contain one or more double bonds, but none of the rings has a completely conjugated $\pi$-electron system. The spiro heterocyclyl is preferably a 6 to 14 membered spiro heterocyclyl, and more preferably a 7 to 10 membered spiro heterocyclyl. According to the number of the spiro atoms shared between the rings, the spiro heterocyclyl is divided into a mono-spiro heterocyclyl, di-spiro heterocyclyl, or poly-spiro heterocyclyl, and the spiro heterocyclyl is preferably a mono-spiro heterocyclyl or di-spiro heterocyclyl, and more preferably a 4-membered/4-membered, 4-membered/5-membered, 4-membered/6-membered, 5-membered/5-membered, or 5-membered/6-membered mono-spiro heterocyclyl. Non-limiting examples of spiro heterocyclyl include:

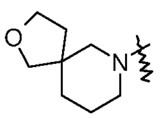 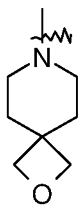 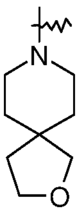 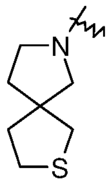 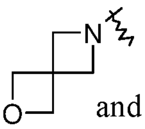 and 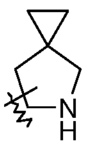.

The term "fused heterocyclyl" refers to a 5 to 20 membered polycyclic heterocyclyl group, wherein each ring in the system shares an adjacent pair of atoms with another ring, wherein one or more rings can contain one or more double bonds, but none of the rings has a completely conjugated $\pi$-electron system, and wherein one or more ring atoms are heteroatoms selected from the group consisting of N, O and $S(O)_m$ (wherein m is an integer of 0 to 2), with the remaining ring atoms being carbon atoms. The fused heterocyclyl is preferably a 6 to 14 membered fused heterocyclyl, and more preferably a 7 to 10 membered fused heterocyclyl. According to the number of membered rings, the fused heterocyclyl can be divided into a bicyclic, tricyclic, tetracyclic or polycyclic fused heterocyclyl, and the fused heterocyclyl is preferably a bicyclic or tricyclic fused heterocyclyl, and more preferably a 5-membered/5-membered or 5-membered/6-membered bicyclic fused heterocyclyl. Non-limiting examples of fused heterocyclyl include:

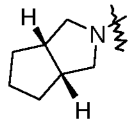 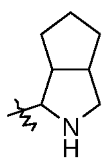 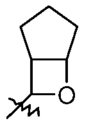 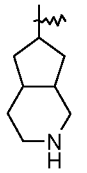 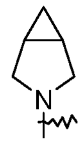 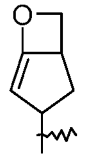 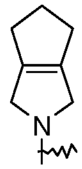 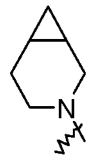

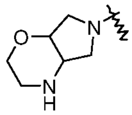 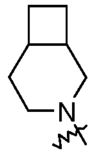 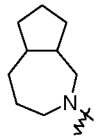 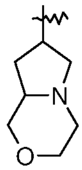 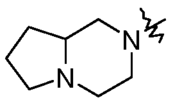 and 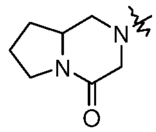 .

The term "bridged heterocyclyl" refers to a 5 to 14 membered polycyclic heterocyclyl group, wherein every two rings in the system share two disconnected atoms, wherein the rings can have one or more double bonds, but none of the rings has a completely conjugated π-electron system, and wherein one or more ring atoms are heteroatoms selected from the group consisting of N, O and $S(O)_m$ (wherein m is an integer of 0 to 2), with the remaining ring atoms being carbon atoms. The bridged heterocyclyl is preferably a 6 to 14 membered bridged heterocyclyl, and more preferably a 7 to 10 membered bridged heterocyclyl. According to the number of membered rings, the bridged heterocyclyl can be divided into a bicyclic, tricyclic, tetracyclic or polycyclic bridged heterocyclyl, and the bridged heterocyclyl is preferably a bicyclic, tricyclic or tetracyclic bridged heterocyclyl, and more preferably a bicyclic or tricyclic bridged heterocyclyl. Non-limiting examples of bridged heterocyclyl include:

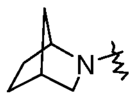 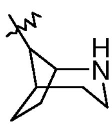 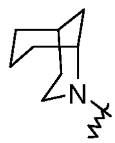 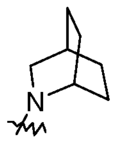

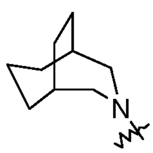 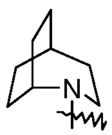 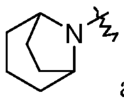 and 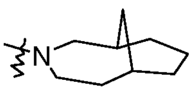

The heterocyclyl (including heterocyclyl, spiro heterocyclyl, fused heterocyclyl and bridged heterocyclyl) ring can be fused to the ring of aryl, heteroaryl or cycloalkyl, wherein the ring bound to the parent structure is heterocyclyl, and non-limiting examples thereof include:

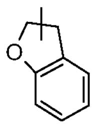 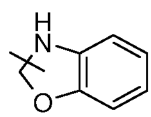 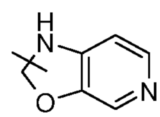 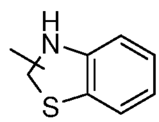

and the like.

The heterocyclyl can be optionally substituted or unsubstituted. When substituted, the substituent group(s) is preferably one or more groups independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, oxo, carboxy and alkoxycarbonyl.

The term "aryl" refers to a 6 to 14 membered all-carbon monocyclic ring or polycyclic fused ring (*i.e.* each ring in the system shares an adjacent pair of carbon atoms with another ring in the system) having a conjugated π-electron system, preferably a 6 to 10 membered aryl, for example, phenyl and naphthyl. The aryl ring can be fused to the ring of heteroaryl, heterocyclyl or cycloalkyl, wherein the ring bound to the parent structure is aryl ring, and non-limiting examples thereof include:

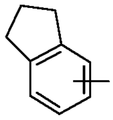 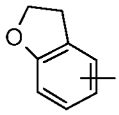 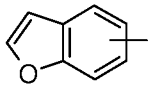 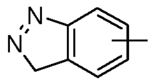 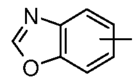 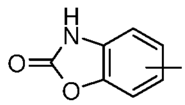 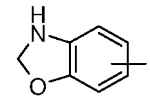

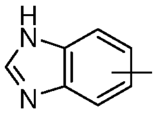 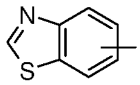 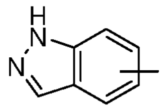 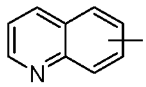 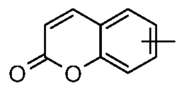 and 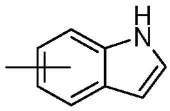 .

The aryl can be substituted or unsubstituted. When substituted, the substituent group(s) is preferably one or more groups independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, carboxy and alkoxycarbonyl.

The term "heteroaryl" refers to a 5 to 14 membered heteroaromatic system having 1 to 4 heteroatoms selected from the group consisting of O, S and N. The heteroaryl is preferably a 5 to 10 membered heteroaryl having 1 to 3 heteroatoms, more preferably a 5 or 6 membered heteroaryl having 1 to 2 heteroatoms; preferably for example, imidazolyl, furyl, thienyl, thiazolyl, pyrazolyl, oxazolyl, pyrrolyl, tetrazolyl, pyridyl, pyrimidinyl, thiadiazolyl, pyrazinyl, pyridazinyl and the like, preferably pyridazinyl and pyridinyl, and more preferably pyridazinyl. The heteroaryl ring can be fused to the ring of aryl, heterocyclyl or cycloalkyl, wherein the ring bound to the parent structure is heteroaryl ring, and non-limiting examples thereof include:

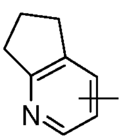 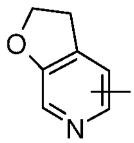 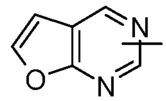 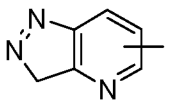 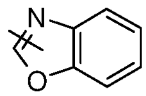 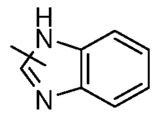

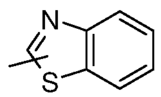 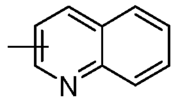 and 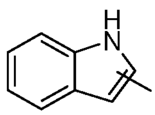 .

The heteroaryl can be optionally substituted or unsubstituted. When substituted, the substituent group(s) is preferably one or more groups independently selected from the group consisting of alkyl, alkenyl, alkynyl, alkoxy, alkylthio, alkylamino, halogen, thiol, hydroxy, oxo, nitro, cyano, cycloalkyl, heterocyclyl, aryl, heteroaryl, cycloalkoxy, heterocycloalkoxy, cycloalkylthio, heterocyclylthio, carboxy and alkoxycarbonyl, and non-limiting examples thereof include

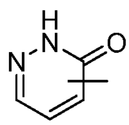 .

The term "hydroxyalkyl" refers to an alkyl group substituted by hydroxy(s), wherein the alkyl is as defined above.

The term "haloalkyl" refers to an alkyl group substituted by one or more halogens, wherein the alkyl is as defined above.

The term "haloalkoxy" refers to an alkoxy group substituted by one or more halogens, wherein the alkoxy is as defined above.

The term "deuterated alkyl" refers to an alkyl group substituted by one or more deuterium atoms, wherein the alkyl is as defined above.

The term "deuterated alkoxy" refers to an alkoxy group substituted by one or more deuterium atoms, wherein the alkoxy is as defined above.

The term "cycloalkyloxy" refers to a -O-cycloalkyl group, wherein the cycloalkyl is as defined above.

The term "hydroxy" refers to an -OH group.

The term "halogen" refers to fluorine, chlorine, bromine or iodine.

The term "amino" refers to a $-NH_2$ group.

The term "cyano" refers to a -CN group.

The term "nitro" refers to a $-NO_2$ group.

The term "carboxy" refers to a -C(O)OH group.

The term "alkoxycarbonyl" refers to a -C(O)O(alkyl) or -C(O)O(cycloalkyl) group, wherein the alkyl and cycloalkyl are as defined above. The term "carboxylate" refers to a $-C(O)O^-Q^+$ group, where $Q^+$ is a pharmaceutically acceptable monovalent positive ion (for example, a metal ion or an ammonium ion, etc.).

The term "acyl halide" refers to a compound containing a -C(O)-halogen group.

The term "pharmaceutically acceptable monovalent cation" ($Q^+$) includes, for example, $N(R^y)_4$ (where $R^y$ is H or $C_1$-$C_4$ alkyl), alkali metal ions (such as potassium, sodium and lithium ions), dicyclohexylamine ion, and N-methyl D-reduced glucosamine ion.

The term "pharmaceutically acceptable divalent cation" ($W^{2+}$) includes alkaline earth metal ions, such as calcium and magnesium ions, and divalent aluminum ions. It also includes amino acid cations, such as monovalent or divalent ions of arginine, lysine, ornithine, etc. A pharmaceutically acceptable divalent cation ($W^{2+}$) can be replaced by two pharmaceutically acceptable monovalent cations ($Q^+$).

The compound of the present disclosure can also comprise isotopic derivatives thereof. The term "isotopic derivatives" refers to compounds that differ in structure only in the presence of one or more isotopically enriched atoms. For example, a compound having the structure of the present disclosure except replacing hydrogen with "deuterium" or "tritium", or replacing fluorine with an $^{18}F$-fluorine labeling ($^{18}F$ isotope), or replacing carbon with $^{11}C$-, $^{13}C$-, or $^{14}C$-enriched carbon ($^{11}C$-, $^{13}C$-, or $^{14}C$-carbon labeling; $^{11}C$-, $^{13}C$-, or $^{14}C$-isotope) is within the scope of the present disclosure. Such compounds can be used, for example, as analytical tools or probes in biological assays, or as tracers for in vivo diagnostic imaging of disease, or as tracers for pharmacodynamics, pharmacokinetics or receptor studies.

The present disclosure also comprises the compounds of formula (I) in various deuterated forms. Each of the available hydrogen atoms attached to the carbon atom can be independently replaced by a deuterium atom. Those skilled in the art can synthesize a compound of formula (I) in a deuterated form with reference to the relevant literatures. The compound of formula (I) in deuterated form can be prepared by employing commercially available deuterated raw materials, or they can be synthesized by conventional techniques with deuterated reagents including, but not limited to, deuterated borane, trideuterated borane in tetrahydrofuran, deuterated lithium aluminum hydride, deuterated iodoethane, deuterated iodomethane and the like.

"Optional" or "optionally" means that the event or circumstance described subsequently can, but need not, occur, and such a description includes the situation in which the event or circumstance does or does not occur. For example, "the heterocyclyl optionally substituted by an alkyl" means that an alkyl group can be, but need not be, present, and such a description includes the situation of the heterocyclyl being substituted by an alkyl and the heterocyclyl being not substituted by an alkyl.

"Substituted" refers to one or more hydrogen atoms in a group, preferably up to 5, and more preferably 1 to 3 hydrogen atoms, independently substituted by a corresponding number of substituents. It goes without saying that the substituents only exist in their possible chemical position. The person skilled in the art is able to determine whether the substitution is possible or impossible by experiments or theory without excessive effort. For example, the combination of amino or hydroxy having free hydrogen and carbon atoms having unsaturated bonds (such as olefinic) may be unstable.

The term "pharmaceutical composition" refers to a mixture of one or more of the compounds described herein or physiologically/pharmaceutically acceptable salts or prodrugs thereof with other chemical components, and other components such as physiologically/pharmaceutically acceptable carriers and excipients. The purpose of the pharmaceutical composition is to facilitate administration of a compound to an organism, which is conducive to the absorption of the active ingredient so as to show biological activity.

A "pharmaceutically acceptable salt" refers to a salt of the compound of the present disclosure, which is safe and effective in mammals and has the desired biological activity.

Synthesis Method of the Compound of the Present Disclosure

In order to achieve the object of the present disclosure, the present disclosure applies the following technical solutions:

Scheme I

A method for preparing the compound of formula (I) or the tautomer, mesomer, racemate, enantiomer, diastereomer thereof, or mixture thereof, or the pharmaceutically acceptable salt thereof according to the present disclosure, comprising the following steps of:

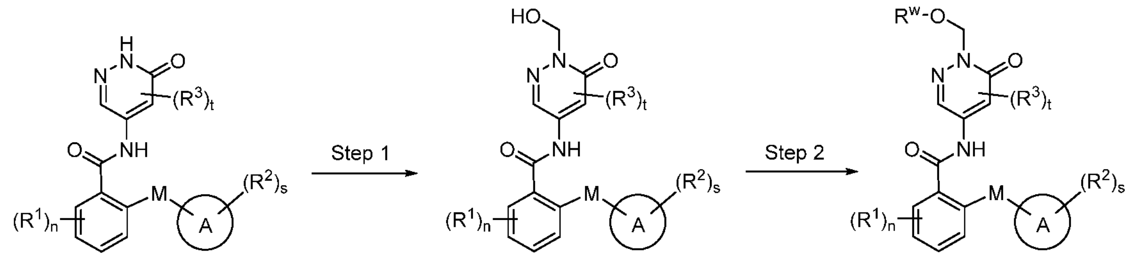


( IB ) ( IA ) ( I )

in Step 1, a compound of formula (IB) and formaldehyde solution are heated to reflux in a suitable solvent (preferably methanol) to obtain a compound of formula (IA);

in Step 2, the compound of formula (IA) is reacted with $R^w$-X,

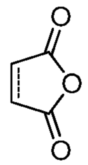

or sulfur trioxide pyridine optionally under an alkaline condition to obtain the compound of formula (I); wherein:

$R^w$ is $-C(O)R^6$ or $-S(O)_2OH$;
X is a halogen or hydroxy;
===== is a single bond or double bond; and
ring A, M, $R^1$, $R^2$, $R^3$, $R^6$, n, s and t are as defined in formula (I).

The reagent that provides an alkaline condition includes organic bases and inorganic bases. The organic bases include, but are not limited to, pyridine, hexahydropyridine, 4-dimethylaminopyridine, triethylamine, N,N-diisopropylethylamine, *n*-butyllithium, lithium diisopropylamide, potassium acetate, sodium *tert*-butoxide and potassium *tert*-butoxide. The inorganic bases include, but are not limited to, sodium hydride, potassium phosphate, sodium carbonate, sodium acetate, potassium carbonate, potassium acetate, cesium carbonate, sodium hydroxide and lithium hydroxide. The reagent that provides an alkaline condition is preferably 4-dimethylaminopyridine or N,N-diisopropylethylamine.

The above reactions are preferably carried out in a solvent. The solvent used includes, but is not limited to, acetic acid, trifluoroacetic acid, methanol, ethanol, toluene, tetrahydrofuran, dichloromethane, petroleum ether, ethyl acetate, *n*-hexane, dimethyl sulfoxide, 1,4-dioxane, water, *N,N*-dimethylformamide and mixtures thereof.

Scheme II

A method for preparing the compound of formula (II) or the tautomer, mesomer, racemate, enantiomer, diastereomer thereof, or mixture thereof, or the pharmaceutically acceptable salt thereof according to the present disclosure, comprising the following steps of:

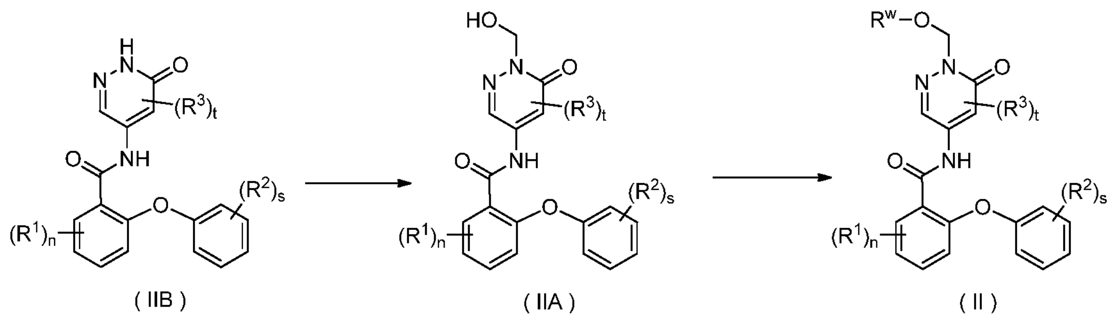

( IIB ) ( IIA ) ( II )

in Step 1, a compound of formula (IIB) and formaldehyde solution are heated to reflux in a suitable solvent (preferably methanol) to obtain a compound of formula (IIA);

in Step 2, the compound of formula (IIA) is reacted with $R^w$-X,

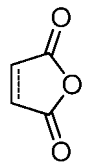

or sulfur trioxide pyridine optionally under an alkaline condition to obtain the compound of formula (II); wherein:

$R^w$ is -C(O)$R^6$ or -$S(O)_2OH$;
X is a halogen or hydroxy;
===== is a single bond or double bond; and
$R^1$, $R^2$, $R^3$, $R^6$, n, s and t are as defined in formula (II).

The reagent that provides an alkaline condition includes organic bases and inorganic bases. The organic bases include, but are not limited to, pyridine, hexahydropyridine, 4-dimethylaminopyridine, triethylamine, N,N-diisopropylethylamine, *n*-butyllithium, lithium diisopropylamide, potassium acetate, sodium *tert*-butoxide and potassium *tert*-butoxide. The inorganic bases include, but are not limited to, sodium hydride, potassium phosphate, sodium carbonate, sodium acetate, potassium carbonate, potassium acetate, cesium carbonate, sodium hydroxide and lithium hydroxide. The reagent that provides an alkaline condition is preferably 4-dimethylaminopyridine or N,N-diisopropylethylamine.

The above reactions are preferably carried out in a solvent. The solvent used includes, but is not limited to, acetic acid, trifluoroacetic acid, methanol, ethanol, toluene, tetrahydrofuran, dichloromethane, petroleum ether, ethyl acetate, *n*-hexane, dimethyl sulfoxide, 1,4-dioxane, water, *N,N*-dimethylformamide and mixtures thereof.

Scheme III

A method for preparing the compound of formula (IIaa) or the tautomer, mesomer, racemate, enantiomer, diastereomer thereof, or mixture thereof, or the pharmaceutically acceptable salt thereof according to the present disclosure, comprising the following steps of:

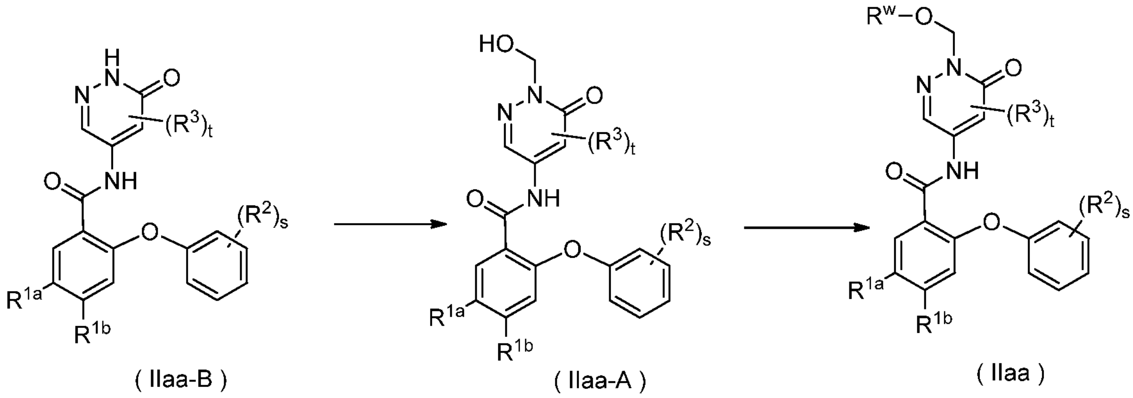

( IIaa-B ) ( IIaa-A ) ( IIaa )

in Step 1, a compound of formula (IIaa-B) and formaldehyde solution are heated to reflux in a suitable solvent (preferably methanol) to obtain a compound of formula (IIaa-A);

in Step 2, the compound of formula (IIaa-A) is reacted with $R^w$-X,

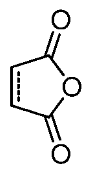

or sulfur trioxide pyridine optionally under an alkaline condition to obtain the compound of formula (IIaa); wherein:

$R^w$ is -C(O)$R^6$ or -S(O)$_2$OH;
X is a halogen or hydroxy;
===== is a single bond or double bond; and
$R^{1a}$, $R^{1b}$, $R^2$, $R^3$, $R^6$, s and t are as defined in formula (IIaa).

The reagent that provides an alkaline condition includes organic bases and inorganic bases. The organic bases include, but are not limited to, pyridine, hexahydropyridine, 4-dimethylaminopyridine, triethylamine, N,N-diisopropylethylamine, *n*-butyllithium, lithium diisopropylamide, potassium acetate, sodium *tert*-butoxide and potassium *tert*-butoxide. The inorganic bases include, but are not limited to, sodium hydride, potassium phosphate, sodium carbonate, sodium acetate, potassium carbonate, potassium acetate, cesium carbonate, sodium hydroxide and lithium hydroxide. The reagent that provides an alkaline condition is preferably 4-dimethylaminopyridine or N,N-diisopropylethylamine.

The above reactions are preferably carried out in a solvent. The solvent used includes, but is not limited to, acetic acid, trifluoroacetic acid, methanol, ethanol, toluene, tetrahydrofuran, dichloromethane, petroleum ether, ethyl acetate, *n*-hexane, dimethyl sulfoxide, 1,4-dioxane, water, *N,N*-dimethylformamide and mixtures thereof.

Scheme IV

A method for preparing the compound of formula (III) or the tautomer, mesomer, racemate, enantiomer, diastereomer thereof, or mixture thereof, or the pharmaceutically acceptable salt thereof according to the present disclosure, comprising the following steps of:

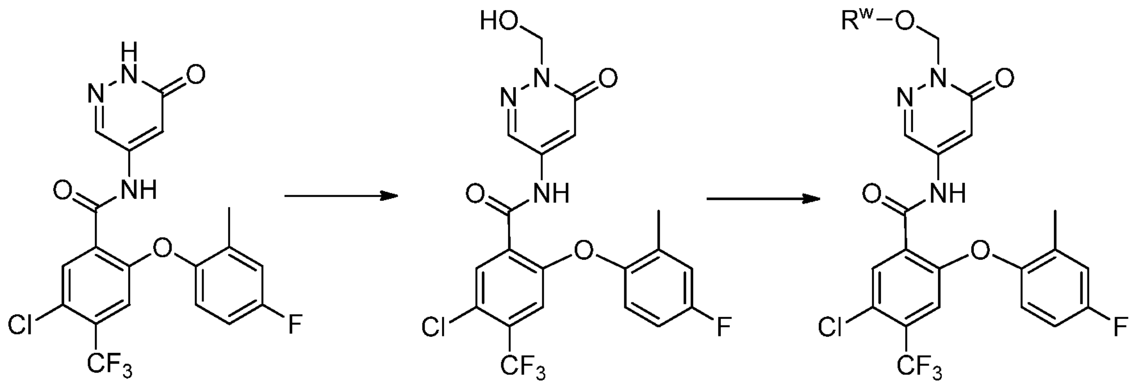

1h 1 ( III )

in Step 1, compound 1h and formaldehyde solution are heated to reflux in a suitable solvent (preferably methanol) to obtain compound 1;

in Step 2, the compound 1 is reacted with $R^w$-X,

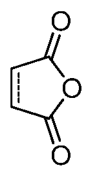

or sulfur trioxide pyridine optionally under an alkaline condition to obtain the compound of formula (III); wherein:

$R^w$ is $-C(O)R^6$ or $-S(O)_2OH$;
X is a halogen or hydroxy;
===== is a single bond or double bond; and
$R^6$ is as defined in formula (III).

The reagent that provides an alkaline condition includes organic bases and inorganic bases. The organic bases include, but are not limited to, pyridine, hexahydropyridine, 4-dimethylaminopyridine, triethylamine, N,N-diisopropylethylamine, *n*-butyllithium, lithium diisopropylamide, potassium acetate, sodium *tert*-butoxide and potassium *tert*-butoxide. The inorganic bases include, but are not limited to, sodium hydride, potassium phosphate, sodium carbonate, sodium acetate, potassium carbonate, potassium acetate, cesium carbonate, sodium hydroxide and lithium hydroxide. The reagent that provides an alkaline condition is preferably 4-dimethylaminopyridine or N,N-diisopropylethylamine.

The above reactions are preferably carried out in a solvent. The solvent used includes, but is not limited to, acetic acid, trifluoroacetic acid, methanol, ethanol, toluene, tetrahydrofuran, dichloromethane, petroleum ether, ethyl acetate, *n*-hexane, dimethyl sulfoxide, 1,4-dioxane, water, *N,N*-dimethylformamide and mixtures thereof.

Scheme V

A method for preparing the compound of formula (IV) or the tautomer, mesomer, racemate, enantiomer, diastereomer thereof, or mixture thereof, or the pharmaceutically acceptable salt thereof according to the present disclosure, comprising the following steps of:

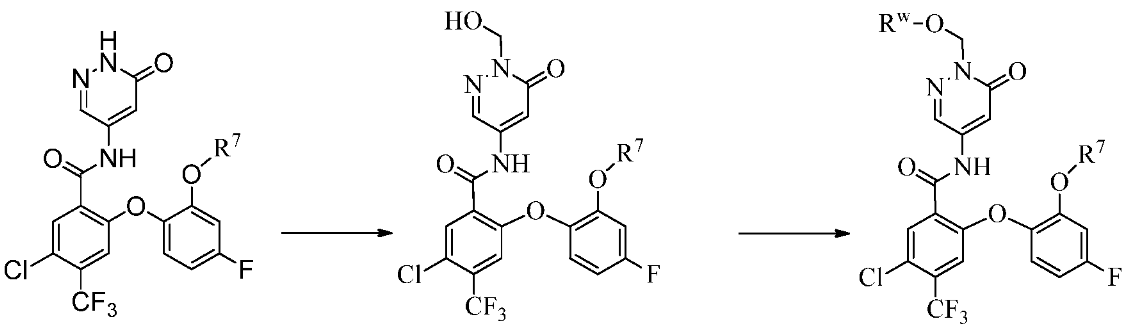

( IVB ) ( IVA ) ( IV )

in Step 1, a compound of formula (IVB) and formaldehyde solution are heated to reflux in a suitable solvent (preferably methanol) to obtain a compound of formula (IVA);

in Step 2, the compound of formula (IVA) is reacted with $R^w$-X,

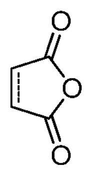

or sulfur trioxide pyridine optionally under an alkaline condition to obtain the compound of formula (IV); wherein:

$R^w$ is $-C(O)R^6$ or $-S(O)_2OH$;
X is a halogen or hydroxy;
===== is a single bond or double bond; and
$R^6$ and $R^7$ are as defined in formula (IV).

The reagent that provides an alkaline condition includes organic bases and inorganic bases. The organic bases

include, but are not limited to, pyridine, hexahydropyridine, 4-dimethylaminopyridine, triethylamine, N,N-diisopropylethylamine, *n*-butyllithium, lithium diisopropylamide, potassium acetate, sodium *tert*-butoxide and potassium *tert*-butoxide. The inorganic bases include, but are not limited to, sodium hydride, potassium phosphate, sodium carbonate, sodium acetate, potassium carbonate, potassium acetate, cesium carbonate, sodium hydroxide and lithium hydroxide. The reagent that provides an alkaline condition is preferably 4-dimethylaminopyridine or N,N-diisopropylethylamine.

The above reactions are preferably carried out in a solvent. The solvent used includes, but is not limited to, acetic acid, trifluoroacetic acid, methanol, ethanol, toluene, tetrahydrofuran, dichloromethane, petroleum ether, ethyl acetate, *n*-hexane, dimethyl sulfoxide, 1,4-dioxane, water, *N,N*-dimethylformamide and mixtures thereof.

## DETAILED DESCRIPTION

The present disclosure will be further described with reference to the following examples, but the examples should not be considered as limiting the scope of the present disclosure.

## EXAMPLES

The structures of the compounds were identified by nuclear magnetic resonance (NMR) and/or mass spectrometry (MS). NMR shifts (δ) are given in $10^{-6}$ (ppm). NMR is determined by a Bruker AVANCE-400 machine. The solvents for determination are deuterated-dimethyl sulfoxide (DMSO-$d_6$), deuterated-chloroform ($CDCl_3$) and deuterated-methanol ($CD_3OD$), and the internal standard is tetramethylsilane (TMS).

MS was determined by an Agilent 1200 /1290 DAD- 6110/6120 Quadrupole MS liquid chromatograph/mass spectrometer (manufacturer: Agilent, MS model: 6110/6120 Quadrupole MS), waters ACQuity UPLC-QD/SQD (manufacturer: waters, MS model: waters ACQuity Qda Detector/waters SQ Detector), THERMO Ultimate 3000-Q Exactive (manufacturer: THERMO, MS model: THERMO Q Exactive).

High performance liquid chromatography (HPLC) was determined on an Agilent HPLC 1200DAD, Agilent HPLC 1200VWD and Waters HPLC e2695-2489 high pressure liquid chromatograph.

Chiral HPLC was determined on an Agilent 1260 DAD high performance liquid chromatograph.

Preparative chromatography was carried out on Waters 2545-2767, Waters 2767-SQ Detecor2, Shimadzu LC-20AP and Gilson GX-281 preparative chromatographs.

Chiral preparation was carried out on a Shimadzu LC-20AP preparative chromatograph.

CombiFlash rapid preparation instrument used was Combiflash Rf200 (TELEDYNE ISCO).

Yantai Huanghai HSGF254 or Qingdao GF254 silica gel plate was used as the thin-layer silica gel chromatography (TLC) plate. The dimension of the silica gel plate used in TLC was 0.15 mm to 0.2 mm, and the dimension of the silica gel plate used in product purification was 0.4 mm to 0.5 mm.

Yantai Huanghai 200 to 300 mesh silica gel was generally used as a carrier for silica gel column chromatography.

The average kinase inhibition rates and $IC_{50}$ values were determined by a NovoStar microplate reader (BMG Co., Germany).

The known starting materials of the present disclosure can be prepared by the known methods in the art, or can be purchased from ABCR GmbH & Co. KG Acros Organics, Aldrich Chemical Company, Accela ChemBio Inc., Dari Chemical Company etc.

Unless otherwise stated, the reactions were carried out under argon atmosphere or nitrogen atmosphere.

"Argon atmosphere" or "nitrogen atmosphere" means that a reaction flask is equipped with an argon or nitrogen balloon (about1 L).

"Hydrogen atmosphere" means that a reaction flask is equipped with a hydrogen balloon (about1 L).

Pressurized hydrogenation reaction was performed on a Parr 3916EKX hydrogenation instrument and a Qinglan QL-500 hydrogen generator or HC2-SS hydrogenation instrument.

In hydrogenation reactions, the reaction system was generally vacuumed and filled with hydrogen, and the above operation was repeated three times.

CEM Discover-S 908860 type microwave reactor was used in microwave reactions.

Unless otherwise stated, the solution refers to an aqueous solution.

Unless otherwise stated, the reaction temperature is room temperature from 20°C to 30°C.

The reaction process in the examples was monitored by thin layer chromatography (TLC). The developing solvent used in the reactions, the eluent system in column chromatography and the developing solvent system in thin layer chromatography for purification of the compounds included: A: dichloromethane/methanol system, B: *n*-hexane/ethyl acetate system, C: petroleum ether/ethyl acetate system, D: acetone, E: dichloromethane/acetone system, F: ethyl acetate/dichloromethane system, G: ethyl acetate/dichloromethane/*n*-hexane, and H: ethyl acetate/dichloromethane/acetone. The ratio of the volume of the solvent was adjusted according to the polarity of the compounds, and a small quantity of alkaline reagent such as triethylamine or acidic reagent such as acetic acid could also be added for

adjustment.

Example 1

5-Chloro-2-(4-fluoro-2-methylphenoxy)-*N*-(1-(hydroxymethyl)-6-oxo-1,6-dihydropyrid azin-4-yl)-4-(trifluoromethyl)benzamide 1

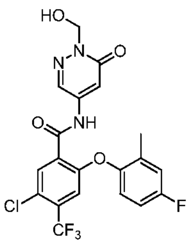

1

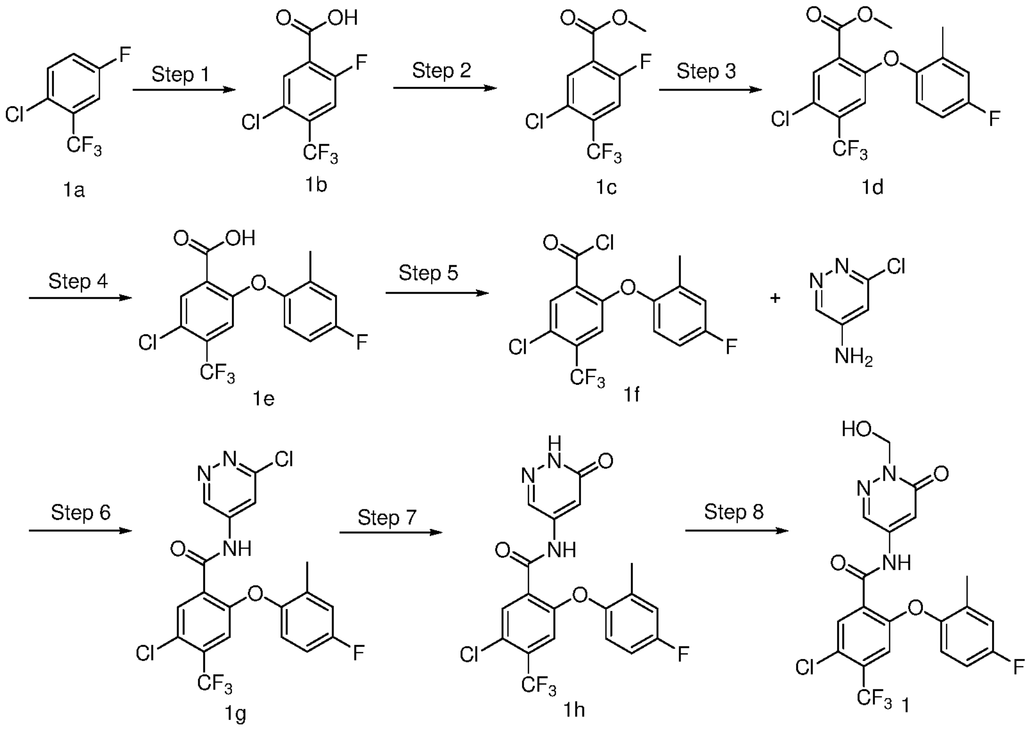


Step 1

5-Chloro-2-fluoro-4-(trifluoromethyl)benzoic acid **1b**

2,2,6,6-Tetramethylpiperidine (19.2 g, 135.93 mmol, Accela ChemBio (Shanghai) Inc.) was added to tetrahydrofuran (200 mL) under an argon atmosphere. The resulting solution was cooled to 0°C, then *n*-butyl lithium (1.6 M, 85.1 mL) was added dropwise within about 45 minutes at a controlled temperature below 3°C. The reaction solution was reacted at 0°C for 1 hour, and then cooled to -78°C. Compound 1-chloro-4-fluoro-2-(trifluoromethyl)benzene **1a** (18 g, 90.66 mmol, Shanghai Titan Scientific Co., Ltd.) was added dropwise, and the reaction solution was reacted for 3 hours. Excess dry ice was added, and the reaction solution was naturally warmed up to 0°C, followed by the addition of 150 mL of ice water. The reaction solution was separated into two phases. The aqueous phase was adjusted to pH 5 to 6 with concentrated hydrochloric acid and extracted with ethyl acetate (50 mL), and the organic phase was concentrated under reduced pressure. The crude product was washed with *n*-hexane (50 mL), then purified by silica gel column chromatography with eluent system A to obtain the title compound **1b** (15 g, yield: 68%).
MS m/z (ESI): 241.1 [M-1].

Step 2

Methyl 5-chloro-2-fluoro-4-(trifluoromethyl)benzoate **1c**

Compound **1b** (5 g, 20.61 mmol) was added to thionyl chloride (49.2 g, 413.55 mmol), and the reaction solution was reacted at 80°C for 2 hours. The reaction solution was concentrated under reduced pressure. The resulting oil was added dropwise to methanol (100 mL), and the reaction solution was reacted at room temperature for 1 hour. The reaction solution was concentrated under reduced pressure, and the resulting residue was purified by silica gel column chromatography with eluent system B to obtain the title compound **1c** (2.78 g, yield: 52%).

Step 3

Methyl 5-chloro-2-(4-fluoro-2-methylphenoxy)-4-(trifluoromethyl)benzoate **1d**

Compound **1c** (2.78 g, 10.83 mmol), 4-fluoro-2-methyl-phenol (1.5 g, 11.89 mmol, Shanghai Bide Pharmatech Ltd.) and cesium carbonate (6 g, 18.41 mmol) were added to *N,N*-dimethylformamide (20 mL), and the reaction solution was reacted at 100°C for 1 hour. The reaction solution was cooled and filtered. The filtrate was concentrated to obtain the target compound **1d** (3.92 g), which was used directly in the next step without purification.
MS m/z (ESI): 363.1 [M+1].

Step 4

5-Chloro-2-(4-fluoro-2-methylphenoxy)-4-(trifluoromethyl)benzoic acid **1e**

The crude compound **1d** (3.92 g, 10.81 mmol) was dissolved in methanol (30 mL), followed by the addition of water (10 mL) and sodium hydroxide (1.3 g, 32.5 mmol), and the reaction solution was reacted for 16 hours. The reaction solution was concentrated, followed by the addition of 10 mL of water, and the pH was adjusted to 1 with concentrated hydrochloric acid. The resulting solution was extracted with ethyl acetate (20 mL×3), and the organic phase was dried over anhydrous sodium sulfate and filtered. The filtrate was concentrated under reduced pressure, and the resulting residue was purified by silica gel column chromatography with eluent system A to obtain the title compound **1e** (3.67 g, yield: 97%).
MS m/z (ESI): 346.8 [M-1].

Step 5

5-Chloro-2-(4-fluoro-2-methylphenoxy)-4-(trifluoromethyl)benzoyl chloride If

Compound **1e** (3.67 g, 10.52 mmol) was added to thionyl chloride (20 g, 168.1 mmol), and the reaction solution was reacted at 80°C for 2 hours. The reaction solution was concentrated to obtain the crude title compound If (3.86 g), which was used directly in the next step without purification.

Step 6

5-Chloro-*N*-(6-chloropyridazin-4-yl)-2-(4-fluoro-2-methylphenoxy)-4-(trifluoromethyl) benzamide **1g**

4-Dimethylaminopyridine (130 mg, 1.05 mmol) and 6-chloropyridazin-4-amine (1.51 g, 11.57 mmol, Pharmablock Sciences (Nanjing), Inc.) were dissolved in pyridine (40 mL), and the resulting solution was dried over molecular sieves. The crude compound If (3.86 g, 10.51 mmol) was added, and the reaction solution was reacted for 16 hours under an argon atmosphere. The reaction solution was concentrated under reduced pressure, and the resulting residue was purified by silica gel column chromatography with eluent system B to obtain the title compound **1g** (1.3 g, yield: 39%).
MS m/z (ESI): 460.0 [M+1].

Step 7

5-Chloro-2-(4-fluoro-2-methylphenoxy)-*N*-(6-oxo-1,6-dihydropyridazin-4-yl)-4-(trifluo romethyl)benzamide **1h**

Compound **1g** (1.3 g, 2.82 mmol) and potassium acetate (555 mg, 5.65 mmol) were added to acetic acid (20 mL), and the reaction solution was reacted at 130°C for 3 hours. The reaction solution was concentrated, and the resulting

residue was purified by silica gel column chromatography with eluent system B to obtain the title compound **1h** (800 mg, yield: 64%).
MS m/z (ESI): 442.0 [M+1].

Step 8

5-Chloro-2-(4-fluoro-2-methylphenoxy)-*N*-(1-(hydroxymethyl)-6-oxo-1,6-dihydropyrid azin-4-yl)-4-(trifluoromethyl)benzamide 1

Compound **1h** (975 mg, 2.21 mmol) was added to methanol (12 mL), followed by the addition of formaldehyde solution (12 g, 147.87 mmol, 37 wt%, Sinopharm Chemical Reagent Co., Ltd.). The reaction solution was heated to reflux for 16 hours under an argon atmosphere. The reaction solution was concentrated under reduced pressure and filtered. The resulting filter cake was washed with water, and dried to obtain the title compound 1 (1.0 g, yield: 96%).

MS m/z (ESI): 472.0 [M+1].
$^1$H NMR (400 MHz, DMSO-$d_6$) δ 11.11 (s, 1H), 8.08 (s, 1H), 7.91 (s, 1H), 7.10-7.22 (m, 2H), 7.04-7.10 (m, 3H), 6.66 (t, 1H), 5.23 (d, 2H), 2.13 (s, 3H).

Example 2

4-((4-(5-Chloro-2-(4-fluoro-2-methylphenoxy)-4-(trifluoromethyl)benzamido)-6-oxopy ridazin-1(6*H*)-yl)methoxy)-4-oxobutanoic acid 2

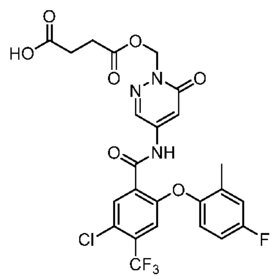

2

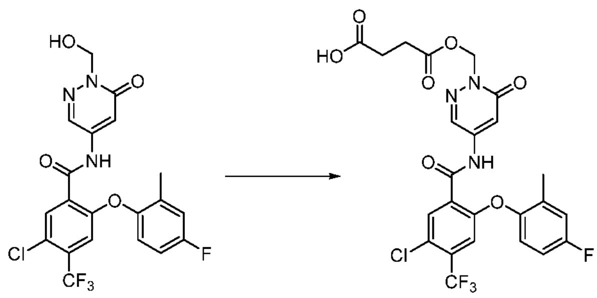

1

2

Compound **1** (990 mg, 2.10 mmol) was added to dichloromethane (20 mL), followed by the addition of succinic anhydride (300 mg, 3.00 mmol, Sinopharm Chemical Reagent Co., Ltd.), 4-dimethylaminopyridine (40 mg, 0.32 mmol) and *N,N*-diisopropylethylamine (600 mg, 4.64 mmol). The reaction solution was reacted at 30°C for 5 hours. The reaction solution was concentrated under reduced pressure, and the resulting residue was purified by preparative high performance liquid chromatography (Waters 2767-SQ Detecor2, eluent system: ammonium acetate, water, acetonitrile) to obtain the title compound **2** (yield: 68%).

MS m/z (ESI): 572.0 [M+1].
$^1$H NMR (400 MHz, DMSO-$d_6$) δ 12.23 (s, 1H), 11.21 (s, 1H), 8.08 (s, 1H), 7.94 (d, 1H), 7.28 (d, 1H), 7.19 (d, 1H), 7.05-7.09 (m, 3H), 5.86 (s, 2H), 2.40-2.53 (m, 4H), 2.13 (s, 3H).

Example 3

(4-(5-Chloro-2-(4-fluoro-2-methylphenoxy)-4-(trifluoromethyl)benzamido)-6-oxopyrid azin-1(6*H*)-yl)methyl isobutyrate **3**

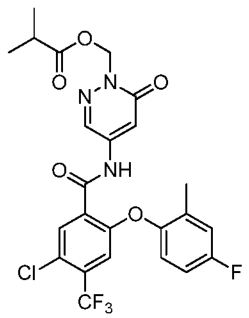

3

In accordance with the synthetic route in Example 2, the starting compound succinic anhydride was replaced with isobutyryl chloride (Sun Chemical Technology (Shanghai) Co., Ltd.), accordingly, the title compound **3** (60 mg, yield: 52%) was prepared.

MS m/z (ESI): 542.1 [M+1].
$^1$H NMR (400 MHz, DMSO-$d_6$) δ 11.21 (s, 1H), 8.08 (s, 1H), 7.94 (s, 1H), 7.27 (s, 1H), 7.19 (d, 1H), 7.05-7.09 (m, 3H), 5.87 (s, 2H), 2.50-2.60 (m, 1H), 2.13 (s, 3H), 1.03 (d, 6H).

Example 4

(4-(5-Chloro-2-(4-fluoro-2-methylphenoxy)-4-(trifluoromethyl)benzamido)-6-oxopyrid azin-1(6*H*)-yl)methyl acetate **4**

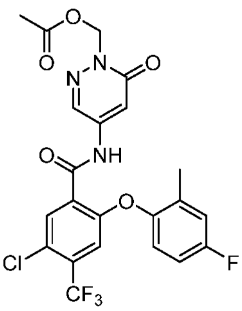

4

In accordance with the synthetic route in Example 2, the starting compound succinic anhydride was replaced with acetyl chloride (Sinopharm Chemical Reagent Co., Ltd.), accordingly, the title compound **4** (60 mg, yield: 55%) was prepared.

MS m/z (ESI): 514.1 [M+1].
$^1$H NMR (400 MHz, DMSO-$d_6$) δ 11.21 (s, 1H), 8.08 (s, 1H), 7.94 (s, 1H), 7.28 (d, 1H), 7.19 (d, 1H), 7.05-7.09 (m, 3H), 5.85 (s, 2H), 2.13 (s, 3H), 2.01 (s, 3H).

Example 5

(4-(5-Chloro-2-(4-fluoro-2-methylphenoxy)-4-(trifluoromethyl)benzamido)-6-oxopyrid azin-1(6*H*)-yl)methyl ethyl carbonate **5**

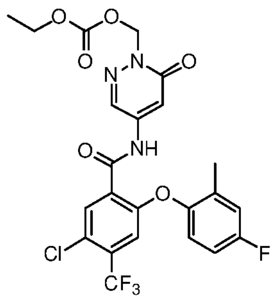

5

In accordance with the synthetic route in Example 2, the starting compound succinic anhydride was replaced with ethyl chloroacetate (Sinopharm Chemical Reagent Co., Ltd.), accordingly, the title compound 5 (15 mg, yield: 13%) was prepared.

MS m/z (ESI): 544.1 [M+1].
$^1$H NMR (400 MHz, DMSO-$d_6$) δ 11.22 (s, 1H), 8.09 (s, 1H), 7.94 (d, 1H), 7.28 (d, 1H), 7.19 (d, 1H), 7.05-7.09 (m, 3H), 5.89 (s, 2H), 4.12 (q, 2H), 2.13 (s, 3H), 1.18 (t, 3H).

Example 6

4-((4-(5-Chloro-2-(4-fluoro-2-methoxyphenoxy)-4-(trifluoromethyl)benzamido)-6-oxop yridazin-1(6*H*)-yl)methoxy)-4-oxobutanoic acid **6**

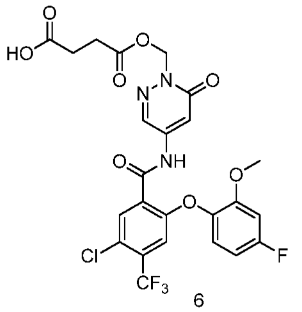

6

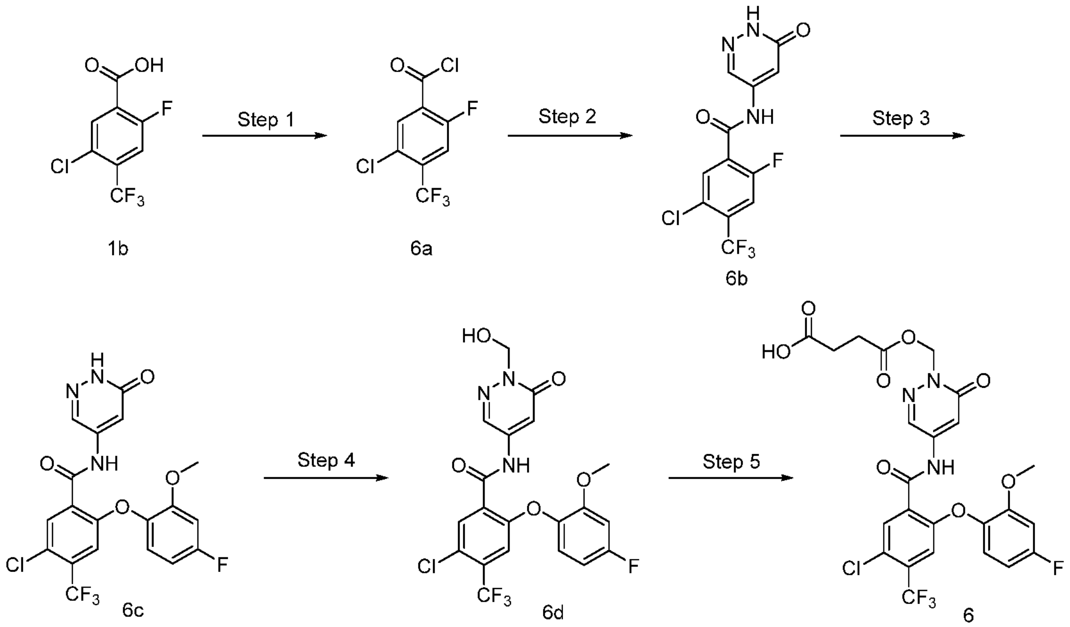


1b 6a 6b 6c 6d 6

Step 1

5-Chloro-2-fluoro-4-(trifluoromethyl)benzoyl chloride 6a

Compound **1b** (5.00 g, 20.6 mmol) was added to 15 mL of thionyl chloride, and the reaction solution was reacted at 80°C for 2 hours. The reaction solution was concentrated under reduced pressure to obtain the crude title compound 6a (5.38 g), which was used directly in the next step without purification.

Step 2

5-Chloro-2-fluoro-*N*-(6-oxo-1,6-dihydropyridazin-4-yl)-4-(trifluoromethyl)benzamide **6b**

5-Aminopyridazin-3-one (3.06 g, 24.8 mmol, prepared according to the method disclosed in Example 386 on page 100 of the description of the patent application "WO2016004417") was dissolved in 40 mL of *N*-methylpyrrolidone. The resulting solution was cooled to 0°C, and sodium hydride (2.06 g, 51.5 mmol, purity: 60%) was slowly add in batches. The reaction solution was stirred at 0°C for 30 minutes. Compound **6a** (5.38 g, 20.6 mmol) was dissolved in 3 mL of *N*-methylpyrrolidone, and the resulting solution was slowly added dropwise to the above reaction solution, which was then stirred at room temperature overnight. The reaction solution containing the title compound **6b** was used directly in the next step without purification.

Step 3

5-Chloro-2-(4-fluoro-2-methoxyphenoxy)-*N*-(6-oxo-1,6-dihydropyiidazin-4-yl)-4-(triflu oromethyl)benzamide **6c**

4-Fluoro-2-methoxyphenol (2.34 g, 16.5 mmol, Tokyo Chemical Industry (Shanghai) Co., Ltd.) and cesium carbonate (6.71 g, 20.6 mmol, Accela ChemBio (Shanghai) Inc.) were added directly to the reaction solution containing

compound **6b.** The reaction solution was reacted at 60°C overnight, and then cooled to room temperature. Ethyl acetate (250 mL) was added, and the reaction solution was washed with water (100 mL×3). The organic phase was dried over anhydrous sodium sulfate and filtered. The filtrate was concentrated under reduced pressure, and the resulting residue was purified by silica gel column chromatography with eluent system B to obtain the title compound **6c** (3.0 g, yield: 32%).

MS m/z (ESI):458.1 [M+1].
$^1$H NMR (400 MHz, DMSO-$d_6$): δ 12.87 (s, 1H), 11.03 (s, 1H), 8.05 (s, 1H), 7.92 (s, 1H), 7.27 (dd, 1H), 7.22 (s, 1H), 7.15 (dd, 1H), 7.00 (s, 1H), 6.87-6.82 (m, 1H), 3.71 (s, 3H).

Step 4

5-Chloro-2-(4-fluoro-2-methoxyphenoxy)-*N*-(1-(hydroxymethyl)-6-oxo-1,6-dihydropyri dazin-4-yl)-4-(trifluoromethyl)benzamide **6d**

Compound **6c** (3.00 g, 6.55 mmol) was added to 30 mL of methanol, followed by the addition of formaldehyde solution (30 mL, 37 wt%, Sinopharm Chemical Reagent Co., Ltd.). The reaction solution was heated to reflux for 16 hours under an argon atmosphere. The reaction solution was concentrated under reduced pressure and filtered. The resulting filter cake was dried to obtain the title compound **6d** (2.90 g, yield: 91%).
MS m/z (ESI): 488.2 [M+1].

Step 5

4-((4-(5-Chloro-2-(4-fluoro-2-methoxyphenoxy)-4-(trifluoromethyl)benzamido)-6-oxop yridazin-1(6*H*)-yl)methoxy)-4-oxobutanoic acid **6**

Compound **6d** (2.90 g, 5.94 mmol) was added to 50 mL of dichloromethane, followed by the addition of succinic anhydride (893 mg, 8.92 mmol, Sinopharm Chemical Reagent Co., Ltd.), 4-dimethylaminopyridine (110 mg, 0.89 mmol) and *N,N*-diisopropylethylamine (1.54 g, 11.92 mmol). The reaction solution was reacted at 30°C overnight. The reaction solution was concentrated under reduced pressure, and the resulting residue was purified by preparative high performance liquid chromatography (Waters 2767-SQ Detecor2, eluent system: ammonium acetate, water, acetonitrile) to obtain the title compound **6** (2.8 g, yield: 80%).

MS m/z (ESI): 588.1 [M+1].
1H NMR (400 MHz, DMSO-$d_6$) δ 12.22 (s, 1H), 11.16 (s, 1H), 8.03 (s, 1H), 7.97 (d, 1H), 7.31 (s, 1H), 7.24 (dd, 1H), 7.11 (dd, 1H), 6.97 (s, 1H), 6.84-7.79 (m,1H), 5.87 (s, 2H), 2.52-2.41 (m,4H), 2.03 (s, 3H).

Example 7

4-((4-(5-Chloro-2-(4-fluoro-2-(methoxy-$d_3$)phenoxy)-4-(trifluoromethyl)benzamido)-6-oxopyridazin-1(6*H*)-yl)methoxy)-4-oxobutanoic acid 7

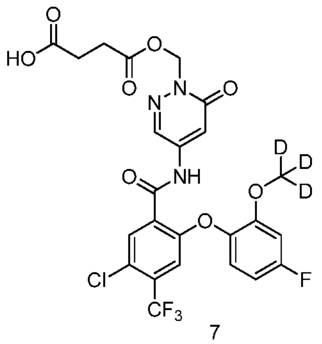

7

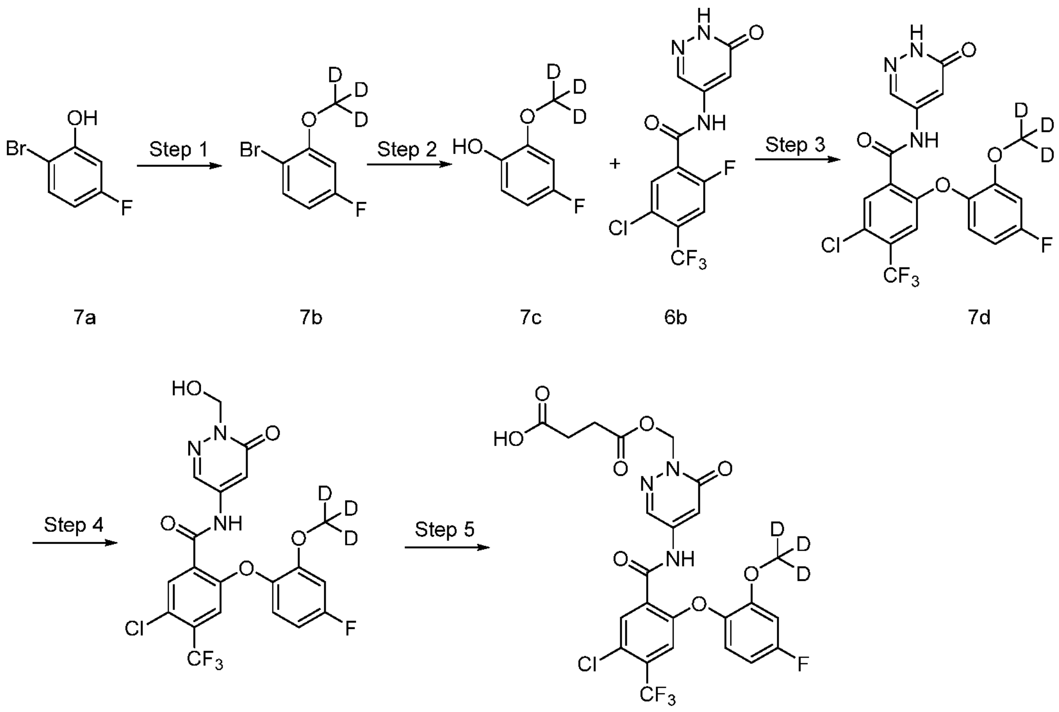


7a 7b 7c 6b 7d

7e 7

Step 1

1-Bromo-4-fluoro-2-(methoxy-*d*$_3$)benzene **7b**

2-Bromo-5-fluorophenol **7a** (1 g, 5.2 mmol, Accela ChemBio (Shanghai) Inc.), deuterated methyl iodide (911 mg, 6.3 mmol, Sun Chemical Technology (Shanghai) Co., Ltd.) and potassium carbonate (1.45 g, 10.5 mmol) were added to *N,N*-dimethylformamide (10 mL). The reaction solution was stirred and reacted for 6 hours. The reaction solution was cooled to room temperature. Ethyl acetate (20 mL) was added, and the reaction solution was washed with water (20 mL×3). The organic phases were combined, dried over anhydrous sodium sulfate and filtered. The filtrate was concentrated under reduced pressure, and the resulting residue was purified by silica gel column chromatography with eluent system A to obtain the title compound 7b (840 mg, yield: 71%).
$^1$H NMR (400 MHz, $CDCl_3$): δ 7.49-7.45 (m, 1H), 6.66-6.57 (m, 2H).

Step 2

4-Fluoro-2-(methoxy-*d*$_3$)phenol **7c**

Compound 7b (840 mg, 4 mmol) and triisopropyl borate (987 mg, 5.25 mmol, Shanghai Titan Scientific Co., Ltd.) were added to a mixed solution of tetrahydrofuran/toluene (150 mL/30 mL). The air in the reaction flask was replaced with argon. The reaction solution was cooled to -78°C, then *n*-butyl lithium (1.6 M, 3.8 mL, 6.1 mmol) was slowly added dropwise within 20 minutes. The reaction solution was naturally warmed up to room temperature and stirred overnight.

The reaction solution was cooled to 0°C in an ice bath. Methanol (50 mL) was added, and hydrogen peroxide (30 wt%, 10 mL) and 10% sodium hydroxide solution (40 mL) were added dropwise. The reaction solution was stirred at room temperature for 1 hour. Saturated sodium thiosulfate solution (50 mL) was slowly added dropwise, and the reaction solution was extracted with ethyl acetate (200 mL×3). The organic phase was washed with saturated sodium bicarbonate solution (150 mL), dried over anhydrous sodium sulfate and filtered. The filtrate was concentrated under reduced pressure, and the resulting residue was purified by silica gel column chromatography with eluent system B to obtain the title compound 7c (570 mg, yield: 97%).

MS m/z (ESI): 144.0 [M-1].
$^1$H NMR (400 MHz, DMSO-$d_6$): $\delta$ 8.89 (s, 1H), 6.85-6.82 (m, 1H), 6.76-6.72 (m, 1H), 6.59-6.54 (m, 1H).

Step 3

5-Chloro-2-(4-fluoro-2-(methoxy-$d_3$)phenoxy)-*N*-(6-oxo-1,6-dihydropyridazin-4-yl)-4-( trifluoromethyl)benzamide **7d**

Compound **6b** (1 g, 2.98 mmol), compound **7c** (433 mg, 2.98 mmol) and cesium carbonate (1.02 g, 3.13 mmol, Accela ChemBio (Shanghai) Inc.) were added to *N*-methylpyrrolidone (10 mL). The reaction solution was reacted at 80°C for 3 hours, and cooled to room temperature. Ethyl acetate (20 mL) was added, and the reaction solution was washed with water (10 mL×3). The organic phases were combined, dried over anhydrous sodium sulfate and filtered. The filtrate was concentrated under reduced pressure, and the resulting residue was purified by silica gel column chromatography with eluent system B to obtain the title compound **7d** (280 mg, yield: 20%).

MS m/z (ESI):461.0 [M-1].
$^1$H NMR (400 MHz, DMSO-$d_6$): $\delta$ 12.87 (s, 1H), 11.03 (s, 1H), 8.06 (s, 1H), 7.93 (d, 1H), 7.29-7.23 (m, 2H), 7.16-7.13 (m, 1H), 7.01 (s, 1H), 6.88-6.83 (m, 1H).

Step 4

5-Chloro-2-(4-fluoro-2-(methoxy-$d_3$)phenoxy)-*N*-(1-(hydroxymethyl)-6-oxo-1,6-dihydr opyridazin-4-yl)-4-(trifluoromethyl)benzamide **7e**

Compound **7d** (6.1 g, 13.2 mmol) was added to 60 mL of methanol, followed by the addition of formaldehyde solution (60 mL, 37 wt%, Sinopharm Chemical Reagent Co., Ltd.). The reaction solution was heated to reflux for 16 hours under an argon atmosphere. The reaction solution was concentrated under reduced pressure and filtered. The resulting filter cake was dried to obtain the title compound **7e** (5.6 g, yield: 86%).

MS m/z (ESI): 491.2 [M+1].

Step 5

4-((4-(5-Chloro-2-(4-fluoro-2-(methoxy-$d_3$)phenoxy)-4-(trifluoromethyl)benzamido)-6-oxopyridazin-1(6*H*)-yl)methoxy)-4-oxobutanoic acid **7**

Compound **7e** (3.43 g, 7 mmol) was added to 80 mL of dichloromethane, followed by the addition of succinic anhydride (1.05 g, 10.5 mmol, Sinopharm Chemical Reagent Co., Ltd.), 4-dimethylaminopyridine (1.09 g, 8.8 mmol) and *N,N*-diisopropylethylamine (1.81 g, 14 mmol). The reaction solution was reacted at 30°C overnight. The reaction solution was concentrated under reduced pressure, and the resulting residue was purified by preparative high performance liquid chromatography (Waters 2767-SQ Detecor2, eluent system: ammonium acetate, water, acetonitrile) to obtain the title compound 7 (2.7 g, yield: 65%).

MS m/z (ESI): 589.0 [M-1], 591.0 [M+1].
$^1$H NMR (400 MHz, DMSO-$d_6$) $\delta$ 12.21 (s, 1H), 11.93 (s, 1H), 8.07 (s, 1H), 8.02 (d, 1H), 7.34 (d, 1H), 7.30-7.26 (m, 1H), 7.16-7.13 (m, 1H), 7.01 (s, 1H), 6.88-6.83 (m, 1H), 5.91 (s, 2H), 2.67-2.46 (m, 4H).

Example 8

(4-(5-Chloro-2-(4-fluoro-2-methylphenoxy)-4-(trifluoromethyl)benzamido)-6-oxopyrid azin-1(6*H*)-yl)methyl hydrogen sulfate **8**

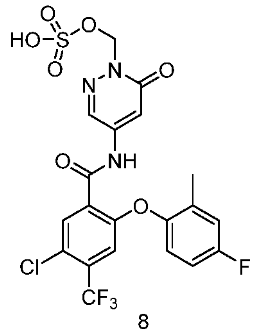

8

In accordance with the synthetic route in Example 2, the starting compound succinic anhydride was replaced with sulfur trioxide pyridine (Accela ChemBio (Shanghai) Inc.), accordingly, the title compound **8** (30 mg) was prepared.

MS m/z (ESI): 550.0 [M-1].
$^1$H NMR (400 MHz, $CD_3OD$) δ 7.98-7.97 (m, 2H), 7.52-7.51 (m, 1H), 7.12-7.09 (m, 1H),7.06-6.97 (m, 3H), 5.88(s, 2H), 2.21(s, 3H).

Example 9

(*E*)-4-((4-(5-Chloro-2-(4-fluoro-2-methylphenoxy)-4-(trifluoromethyl)benzamido)-6-ox opyridazin-1(6*H*)-yl)methoxy)-4-oxobut-2-enoic acid **9**

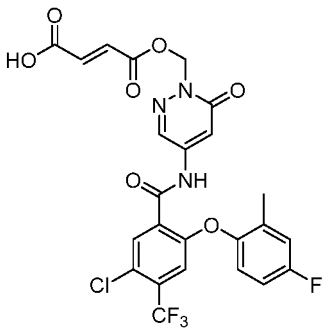

9

In accordance with the synthetic route in Example 2, the starting compound succinic anhydride was replaced with *trans*-butenedioic acid (Accela ChemBio (Shanghai) Inc.), accordingly, the title compound **9** (10 mg) was prepared.

MS m/z (ESI): 570.1 [M+1].
$^1$H NMR (400 MHz, DMSO-$d_6$) δ 12.22 (s, 1H), 8.08 (s, 1H),7.96 (s, 1H), 7.29 (s, 1H), 7.19 (d, 1H), 7.00-7.13(m, 3H), 6.60-6.75 (m, 2H), 6.01 (s, 2H), 2.13 (s, 3H).

Example 10

3-((4-(5-Chloro-2-(4-fluoro-2-methylphenoxy)-4-(trifluoromethyl)benzamido)-6-oxopy ridazin-1(6*H*)-yl)methoxy)-3-oxo-propanoic acid **10**

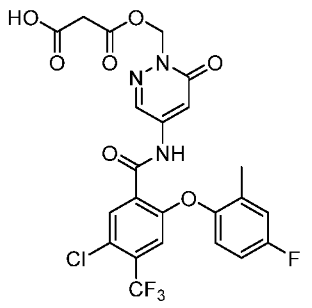

10

In accordance with the synthetic route in Example 2, the starting compound succinic anhydride was replaced with propanedioic acid (Accela ChemBio (Shanghai) Inc.), accordingly, the title compound 10 (13 mg) was prepared.

MS m/z (ESI): 558.0 [M+1].
$^1$H NMR (400 MHz, DMSO-$d_6$) δ 12.22 (s, 1H), 8.09 (s, 1H),7.95 (s, 1H), 7.29 (s, 1H), 7.20 (d, 1H), 7.00-7.13(m, 3H), 5.91 (s, 2H), 3.41 (s, 2H), 2.13 (s, 3H).

Example 11

4-((4-(5-Chloro-2-(2-ethyl-4-fluorophenoxy)-4-(trifluoromethyl)benzamido)-6-oxopyri dazin-1(6*H*)-yl)methoxy)-4-oxobutanoic acid **11**

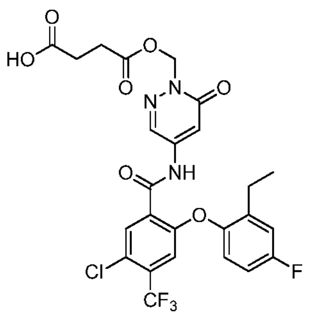

11

In accordance with the synthetic route in Example 6, the starting compound 4-fluoro-2-methoxyphenol of Step 3 was replaced with 2-ethyl-4-fluorophenol (Shanghai Bide Pharmatech Ltd.), accordingly, the title compound **11** (2.3 g) was prepared.

MS m/z (ESI): 584.0 [M-1].
$^1$H NMR (400 MHz, DMSO-$d_6$) δ 12.23 (br, 1H), 11.25 (s, 1H), 8.12 (s, 1H), 7.98-7.98 (dd, 1H), 7.32-7.32 (d, 1H), 7.25-7.23 (d, 1H), 7.13-7.11 (m, 2H), 7.10 (s, 1H), 5.90 (s, 2H), 2.58-2.53 (m, 4H), 2.49-2.46 (m, 2H), 1.10-1.06 (t, 3H).

Example 12

(4-(5-Chloro-2-(4-fluoro-2-(methoxy-*d*$_3$)phenoxy)-4-(trifluoromethyl)benzamido)-6-ox opyridazin-1(6*H*)-yl)methyl hydrogen sulfate **12**

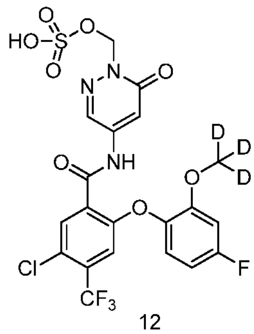

12

In accordance with the synthetic route in Example 7, the starting compound succinic anhydride of Step 5 was replaced with sulfur trioxide pyridine (Accela ChemBio (Shanghai) Inc.), accordingly, the title compound **12** (85 mg) was prepared.

MS m/z (ESI): 568.9 [M-1].
$^1$H NMR (400 MHz, DMSO-*d*$_6$) $\delta$ 8.02 (s, 1H), 7.89 (d, 1H), 7.23-7.19 (m, 2H), 7.09-7.05 (m, 2H), 6.94 (s, 1H), 6.80-6.76 (m, 1H), 5.48 (s, 2H).

Example 13

(*E*)-4-((4-(5-Chloro-2-(4-fluoro-2-(methoxy-*d*$_3$)phenoxy)-4-(trifluoromethyl)benzamido )-6-oxopyiidazin-1(6*H*)-yl)methoxy)-4-oxobut-2-enoic acid **13**

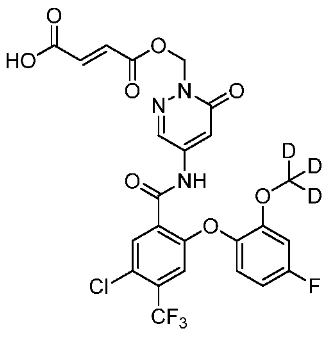

13

In accordance with the synthetic route in Example 7, the starting compound succinic anhydride of Step 5 was replaced with *trans*-butenedioic acid (Accela ChemBio (Shanghai) Inc.), accordingly, the title compound 13 (11 mg) was prepared.

MS m/z (ESI): 587.0 [M-1].
$^1$H NMR (400 MHz, $CD_3OD$) $\delta$ 7.95 (d, 1H), 7.88 (s, 1H), 7.46 (d, 1H), 7.18-7.14 (m, 1H), 6.95 (s, 1H), 6.92-6.89 (m, 1H), 6.79 (d, 1H), 6.71-6.66 (m, 1H), 6.56 (d, 1H), 6.02 (s, 2H).

Physical and Chemical Properties

The present disclosure will be further described with reference to the following test examples, but the test

examples should not be considered as limiting the scope of the present disclosure.

Test Example 1. Solubility of the compound of the present disclosure in PBS solution (pH 7.4) at room temperature

1. Experimental materials

Reagents: dimethyl sulfoxide (analytical grade), ethanol (analytical grade), acetonitrile (chromatographic grade), $NaH_2PO_4$•$2H_2O$ (analytical grade), $Na_2HPO_4$·$12H_2O$ (analytical grade), ammonium acetate (analytical grade), sodium hydroxide, sodium chloride (analytical grade).

Instrument: liquid chromatograph.

2. Experimental procedures

2.1 Formulation of the PBS solution (pH 7.4): 0.57 g of $NaH_2PO_4$•$2H_2O$, 5.55 g of $Na_2HPO_4$•$12H_2O$ and 6.48 g of NaCl were weighed, followed by the addition of ultra-pure water. The pH was adjusted to 7.4±0.05 with 1 M NaOH or 1 M HCl. Water was added until the volume reached 1 L. The PBS solution was stored in a refrigerator at 4°C (the storage life was 6 months).

2.2 Formulation of the solution of the compound in PBS 7.4: An appropriate amount of the test compound was weighed, and dissolved in DMSO or a mixed solution of DMSO: acetonitrile: ethanol (1:1:1) to obtain a 10 mM stock solution of the test compound. 10 μL of the stock solution of the test compound and 990 μL of the PBS solution (pH 7.4) were precisely measured and placed in a 2 mL sample vial and mixed well, and the DMSO concentration of the final solution was 1% (v/v). This solution was formulated in duplicate, shaken on a plate bed at room temperature for 24 hours, and centrifuged at 5000 rpm for 20 minutes. The supernatant was analyzed by the liquid chromatograph.

2.3 Formulation of the reference solution: 10 μL of the stock solution of the test sample (concentration: 10 mM, dissolved in DMSO) and 990 μL of an organic mixed solvent (usually DMSO: acetonitrile: ethanol = 1:1:1) were precisely measured and placed in a 2 mL sample vial, and mixed well to obtain a clear 100 μM sample solution. The solution was filtered through a 0.45 μm organic phase microporous filter membrane, and the filtrate was analyzed by the liquid chromatograph.

3. Data processing

$$\text{Solubility } (\mu M) = \text{peak area of the sample / peak area of the reference * concentration of the reference } (\mu M) \text{ * sample solution dilution factor}$$

The average of two measurements was used as the final solubility.

Table 1 Solubility of the compounds of the present disclosure in PBS solution (pH 7.4)

| Example No. | PBS pH7.4 (μM) |
|---|---|
| 1h | 0.5 |
| 2 | 57.4 |
| 6c | 0.44 |
| 6 | 44.92 |
| 7d | 1.26 |
| 7 | 34.46 |
| 8 | 707.02 |
| 9 | 63.45 |
| 10 | 146.93 |
| 12 | 96.19 |

(continued)

| Example No. | PBS pH7.4 (μM) |
|---|---|
| 13 | 63.59 |

Conclusion: At room temperature, the solubility of compounds 1h, 6c and 7d in the PBS solution (pH 7.4) is poor, while the solubility of the prodrug compounds of the present disclosure in the PBS solution (pH 7.4) is greatly improved.

Biological Assay

Test Example 2. Pharmacokinetics assay of the compounds of the present disclosure in rats

1. Abstract

SD rats were used as test animals. The drug concentration in plasma at different time points was determined by LC/MS/MS method after oral administration of the compounds of Example 2, Example 6 and Example 7 to SD rats. The pharmacokinetic behavior of the compounds of the present disclosure was studied and evaluated in SD rats.

2. Test protocol

2.1 Test compounds

Compounds of Example 2, Example 6 and Example 7.

2.2 Test animals

Forty SD rats (half male and half female, equally divided into ten groups) were purchased from Shanghai Jiesijie Laboratory Animal Co., LTD. (Certificate No.: SCXK(Shanghai)2013-0006).

2.3 Preparation of the test compound

An appropriate amount of the compound of Example 2 was weighed, followed by the addition of 5% of DMSO, 5% of tween 80 and 90% of normal saline successively to obtain a colorless, clear and transparent solution.
An appropriate amount of the compound of Example 2 was weighed, followed by the addition of 0.5% sodium carboxymethyl cellulose (containing 0.5% tween 80) to obtain a white homogeneous suspension.
An appropriate amount of the compound of Example 6 was weighed, and added to 200 mM (5% PVPK30+5% TPGS) $Na_2HPO_4$ solution (pH=9) to obtain a homogeneous suspension.
An appropriate amount of the compound of Example 7 was weighed, and added to 200 mM (5% PVPK30+5% TPGS) $Na_2HPO_4$ solution (pH=9) to obtain a homogeneous suspension.

2.4 Administration

After an overnight fast, the SD rats were intragastrically administered the test compounds. Regarding to the compound of Example 2 (formulation: 5% of DMSO, 5% of tween 80 and 90% of normal saline), the administration dose was 2 mg/kg, the administration volume was 10 mL/kg, and the administration concentration was 0.2 mg/mL. Regarding to the compound of Example 2 (formulation: 0.5% sodium carboxymethyl cellulose (containing 0.5% tween 80)), the administration dose was 10, 30, 100 mg/kg, the administration volume was 10 mL/kg, and the administration concentration was 1, 3, 10 mg/mL. Regarding to the compound of Example 6, the administration dose was 100, 300, 900 mg/kg, the administration volume was 10 mL/kg, and the administration concentration was 10, 30, 90 mg/mL. Regarding to the compound of Example 7, the administration dose was 10, 30, 100 mg/kg, the administration volume was 10 mL/kg, and the administration concentration was 1, 3, 10 mg/mL.

3. Process

After an overnight fast, the SD rats were intragastrically administered the test compounds. 0.2 ml of blood was taken from the orbit before the administration and at 0.5, 1.0, 2.0, 4.0, 6.0, 8.0, 11.0 and 24.0 hours after the administration.

The samples were stored in heparinized tubes, and centrifuged for 10 minutes at 3500 rpm to separate the blood plasma. The plasma samples were stored at -20°C. The SD rats were fed 2 hours after the administration.

The content of the test compound in the plasma of SD rats after administration of the test compound at different concentrations was determined: 25 μL of SD rat plasma at each time point after the administration was taken, followed by the addition of 50 μL of the internal standard camptothecin solution (100 ng/mL) and 200 μL of acetonitrile. The resulting solution was vortex-mixed for 5 minutes, and centrifuged for 10 minutes (3700 rpm). 3.0 μL of the supernatant was taken from the plasma samples of the compound of Example 2 (the group at the administration dose of 2 mg/kg) for LC/MS/MS analysis. 2.0 μL of the supernatant was taken from the plasma samples of the compound of Example 2 (the group at the administration dose of 10, 30, or 100 mg/kg) for LC/MS/MS analysis. 0.2 μL of the supernatant was taken from the plasma samples of the compound of Example 6 for LC/MS/MS analysis. 1 μL of the supernatant was taken from the plasma samples of the compound of Example 7 for LC/MS/MS analysis.

4. Results of pharmacokinetic parameters in SD rats

After the intragastrical administration, the plasma samples were analyzed using LC/MS/MS to determine the concentration of compound 2 and compound 1h, the concentration of compound 6 and compound 6c, as well as the concentration of compound 7 and compound 7d. The compounds of Example 2, Example 6 and Example 7 were not detected in the rats. The following data are the pharmacokinetic data of metabolite compounds 1h, 6c and 7d.

Table 2 Pharmacokinetic parameters of the compounds of the present disclosure in rats

| Test compound | Dose | Compound being analyzed | Plasma concentration | Area under curve | Half-life | Residence time |
|---|---|---|---|---|---|---|
| | | | Cmax (ng/mL) | AUC (ng/mL*h) | Tl/2 (h) | MRT (h) |
| Example 2 | 2 mpk | Compound 1h | 374±69.9 | 5680±2170 | 19.0±14.8 | 28.4±21.5 |
| Example 2 | 10 mpk | Compound 1h | 568±184 | 9336±5628 | -- | -- |
| Example 2 | 30 mpk | Compound 1h | 1196±372 | 19878±9629 | -- | -- |
| Example 2 | 100 mpk | Compound 1h | 2148±794 | 41378±17825 | -- | -- |
| Example 6 | 100 mpk | Compound 6c | 6128±2067 | 67383±36193 | 4.46±1.57 | 7.84±2.11 |
| Example 6 | 300 mpk | Compound 6c | 9573±3392 | 113517±20151 | 19.5±21.6 | 28.6±29.9 |
| Example 6 | 900 mpk | Compound 6c | 7737±1657 | 117947±28070 | 76.4±91.9 | 110±131 |
| Example 7 | 10 mpk | Compound 7d | 945±291.0 | 11027±3704 | 6.66±2.36 | 10.5±2.78 |
| Example 7 | 30 mpk | Compound 7d | 2623±602.1 | 34937±8573 | 10±5.73 | 15±7.56 |
| Example 7 | 100 mpk | Compound 7d | 5178±555 | 92124±12300 | 18.9±7.6 | 28.0±10.3 |

Conclusion: The above research results confirm that in rats, the compound of Example 2 was converted into compound 1h in vivo; the compound of Example 6 was converted into compound 6c in vivo; and the compound of Example 7 was converted into compound 7d in vivo. Moreover, the compounds of the present disclosure are well absorbed, and have a significant pharmacokinetic advantage.

Test Example 3. Determination of the inhibitory activity of the compounds of the present disclosure on $Na_v1.8$

The purpose of the experiment is to investigate the effect of the compounds on $Na_v1.8$ ion channel in an in

vitro experiment, wherein the $Na_V1.8$ ion channel is stably expressed on HEK293 cells. After the $Na_V1.8$ current becomes stable, the $Na_V1.8$ currents before and after the administration of the compound are compared so as to obtain the effect of the compound on the $Na_V1.8$ ion channel.

1. Experimental materials and instruments

1) Patch clamp amplifier: patch clamp PC-505B (WARNER instruments)/MultiClamp 700A (Axon instrument).
2) Digital-to-analog converter: Digidata 1440A (Axon CNS)/Digidata 1550A (Axon instruments).
3) Micro-manipulator: MP-225 (SUTTER instrument).
4) Inverted microscope: TL4 (Olympus).
5) Glass microelectrode puller: PC-10 (NARISHIGE).
6) Microelectrode glass capillary: B12024F (Wuhan Weitan Scientific Instrument Co., Ltd.).
7) Dimethyl sulfoxide (DMSO) D2650 (Sigma-Aldrich).
8) TTX AF3014 (Affix Scientific).

2. Experimental procedures

2.1 Formulation of the compounds

Except for NaOH and KOH used for acid titration and base titration, all the compounds used for formulating the extracellular fluid and intracellular fluid were purchased from Sigma (St. Louis, MO). Extracellular fluid (mM): NaCl, 137; KCl, 4; $CaCl_2$, 1.8; $MgCl_2$, 1; HEPES, 10; glucose, 10; pH 7.4 (NaOH titration). Intracellular fluid (mM): aspartic acid, 140; $MgCl_2$, 2; EGTA, 11; HEPES, 10; pH 7.2 (CsOH titration). All solutions of test compound and control compound contained 1 $\mu$M TTX.

The test compound was dissolved in dimethyl sulfoxide (DMSO) at a stock concentration of 9 mM. The stock solution of the test compound was dissolved in the extracellular fluid on the day of the test and formulated into the required concentration.

2.2 Test process of the manual patch clamp

1) The compound was formulated into solutions with specified concentrations, the solutions were added to the pipelines respectively in order from low to high concentration, and the pipelines were marked.
2) The cell was transferred to the perfusion tank. A positive pressure was applied to the electrode. The tip of the electrode touched the cell. The three-way valve of the air extracting device was adjusted to a three-way state. A negative pressure was applied to the electrode, so that a high-resistance seal was formed between the electrode and the cell. The negative pressure was applied continuously, thereby causing the cell membrane to rupture and forming a current path.
3) After the current for rupturing the cell membrane became stable, perfusion of different concentrations was carried out in sequence. Once the current was stable for at least one minute, perfusion of the next concentration was carried out. The duration of the perfusion of each concentration did not exceed five minutes.
4) The perfusion tank was cleaned. The perfusion tank was rinsed with the drug solutions in order from high to low concentration, and the rinse duration for each concentration of drug solution was 20 seconds. The perfusion tank was finally rinsed with the extracellular fluid for 1 mintue.

2.3 Test voltage equation (resting) and results

The cell was clamped at -80 mV. The cell was depolarized to 10 mV with a square wave lasting 10 milliseconds to obtain the $Na_V1.8$ current. This procedure was repeated every 5 seconds. The maximum current caused by the square wave was measured. After the current became stable, the test compound was perfused. After the response became stable, the blocking intensity was calculated.

3. Data analysis

The data was stored in the computer system for analysis. Data collection and analysis were carried out by

pCLAMP 10 (Molecular Devices, Union City, CA), and the analysis results were reviewed by the administrator. Stable current means that the current changes within a limited range over time. The magnitude of stable current was used to calculate the effect of the compound at the concentration.

The inhibitory activity of the compounds of the present disclosure on $Na_v1.8$ was determined by the above test, and the resulting $IC_{50}$ values are shown in Table 3.

Table 3. $IC_{50}$ of the prodrug compounds and metabolites thereof of the present disclosure on inhibiting the $Na_v1.8$ channel activity

| Example No. | $IC_{50}$ (nM) |
|---|---|
| 1h | 1.3 |
| 2 | 37.6 |
| 6c | 1.3 |
| 6 | 3.9 |
| 7d | 0.54 |
| 7 | 14.5 |

Conclusion: The prodrug compounds and metabolites thereof of the present disclosure have a significant inhibitory effect on the $Na_v1.8$ channel activity.

## Claims

**1.** A compound of formula (I) or a tautomer, mesomer, racemate, enantiomer, diastereomer thereof, or mixture thereof, or a pharmaceutically acceptable salt thereof,

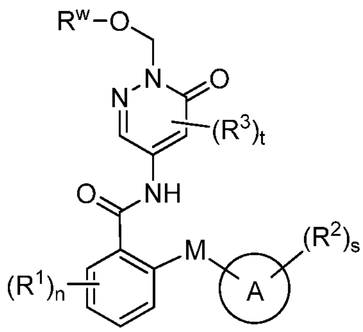

( I )

wherein:

M is selected from the group consisting of O atom, $CR^4R^5$ and S atom;
ring A is an aryl or heteroaryl;
each $R^1$ is identical or different and each is independently selected from the group consisting of hydrogen atom, halogen, alkyl, deuterated alkyl, alkoxy, deuterated alkoxy, haloalkyl, haloalkoxy, cyano, amino, nitro, hydroxy, hydroxyalkyl, cycloalkyl, heterocyclyl, aryl and heteroaryl;
each $R^2$ is identical or different and each is independently selected from the group consisting of hydrogen atom, halogen, alkyl, deuterated alkyl, alkoxy, deuterated alkoxy, haloalkyl, haloalkoxy, cyano, amino, nitro, hydroxy, hydroxyalkyl, cycloalkyl, cycloalkyloxy, heterocyclyl, aryl and heteroaryl, wherein the alkyl, cycloalkyl, heterocyclyl, aryl and heteroaryl are each optionally substituted by one or more substituents selected from the group consisting of alkyl, haloalkyl, halogen, amino, nitro, cyano, hydroxy, alkoxy, haloalkoxy, hydroxyalkyl, cycloalkyl, heterocyclyl, aryl and heteroaryl;
each $R^3$ is identical or different and each is independently selected from the group consisting of hydrogen atom, halogen, alkyl, alkoxy, haloalkyl, cyano, amino, nitro, hydroxy, hydroxyalkyl, cycloalkyl, heterocyclyl, aryl and

heteroaryl;
$R^4$ and $R^5$ are identical or different and are each independently selected from the group consisting of hydrogen atom, deuterium atom, halogen, alkyl, alkoxy, haloalkyl, cyano, amino, nitro, hydroxy, hydroxyalkyl, cycloalkyl, heterocyclyl, aryl and heteroaryl;
$R^w$ is selected from the group consisting of hydrogen atom, alkyl, -C(O)$R^6$, -$S(O)_2OH$, -$S(O)_2O^-Q^+$, -$PO(OH)_2$, -$PO(OH)O^-Q^+$, -$PO(O^-)_2 2Q^+$ and -$PO(O^-)_2W^{2+}$; $Q^+$ is a pharmaceutically acceptable monovalent cation; $W^{2+}$ is a pharmaceutically acceptable divalent cation;
$R^6$ is selected from the group consisting of alkyl, alkoxy, alkenyl, carboxy and carboxylate, wherein the alkyl, alkoxy and alkenyl are each optionally substituted by one or more substituents selected from the group consisting of hydroxy, amino, carboxy and carboxylate;
n is 0, 1, 2, 3 or 4;
s is 0, 1, 2, 3 or 4; and
t is 0, 1 or 2.

**2.** The compound of formula (I) or the tautomer, mesomer, racemate, enantiomer, diastereomer thereof, or mixture thereof, or the pharmaceutically acceptable salt thereof according to claim 1, wherein ring A is a phenyl or pyridyl.

**3.** The compound of formula (I) or the tautomer, mesomer, racemate, enantiomer, diastereomer thereof, or mixture thereof, or the pharmaceutically acceptable salt thereof according to claim 1 or 2, wherein $R^w$ is selected from the group consisting of hydrogen atom, -C(O)-alkyl, -C(O)-alkoxy, -C(O)-alkylene-COOH, -C(O)-alkenylene-COOH, -C(O)-COOH, -$S(O)_2OH$ and -C(O)-alkylene-$NH_2$, wherein the alkyl, alkoxy, alkylene and alkenylene are each optionally substituted by one or more hydroxys; and preferably, $R^w$ is selected from the group consisting of hydrogen atom, -C(O)-$C_{1-6}$ alkyl, -C(O)-$C_{1-6}$ alkoxy, -C(O)-$C_{1-6}$ alkylene-COOH, -C(O)-$C_{2-6}$ alkenylene-COOH, -C(O)-COOH, -$S(O)_2OH$ and -C(O)-$C_{1-6}$ alkylene-$NH_2$, wherein the $C_{1-6}$ alkyl, $C_{1-6}$ alkoxy, $C_{1-6}$ alkylene and $C_{2-6}$ alkenylene are each optionally substituted by one or more hydroxys.

**4.** The compound of formula (I) or the tautomer, mesomer, racemate, enantiomer, diastereomer thereof, or mixture thereof, or the pharmaceutically acceptable salt thereof according to any one of claims 1 to 3, wherein $R^w$ is selected from the group consisting of hydrogen atom, -$C(O)CH_3$, -$C(O)CH(OH)CH_3$, -$C(O)CH(CH_3)_2$, -$C(O)OCH_2CH_3$, -$C(O)CH_2COOH$, -$C(O)CH_2CH_2COOH$, -$C(O)CH(OH)CH_2COOH$, -$C(O)CH_2CH(OH)COOH$, -C(O)CH(OH)CH(OH)COOH, -C(O)-CH=CH-COOH, -C(O)-COOH, -$S(O)_2OH$ and -$C(O)CH_2NH_2$, and preferably selected from the group consisting of -$C(O)CH_2COOH$, -$C(O)CH_2CH_2COOH$, -$C(O)CH(OH)CH_2COOH$, -$C(O)CH_2CH(OH)COOH$, -C(O)CH(OH)CH(OH)COOH, -C(O)-CH=CH-COOH and -C(O)-COOH.

**5.** The compound of formula (I) or the tautomer, mesomer, racemate, enantiomer, diastereomer thereof, or mixture thereof, or the pharmaceutically acceptable salt thereof according to any one of claims 1 to 4, wherein M is an O.

**6.** The compound of formula (I) or the tautomer, mesomer, racemate, enantiomer, diastereomer thereof, or mixture thereof, or the pharmaceutically acceptable salt thereof according to any one of claims 1 to 5, being a compound of formula (II) or a tautomer, mesomer, racemate, enantiomer, diastereomer thereof, or mixture thereof, or a pharmaceutically acceptable salt thereof,

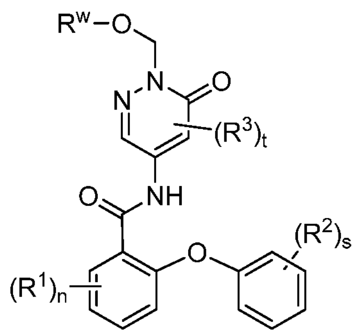

( II )

wherein:
$R^1$, $R^2$, $R^3$, $R^w$, n, s and t are as defined in claim 1.

**7.** The compound of formula (I) or the tautomer, mesomer, racemate, enantiomer, diastereomer thereof, or mixture thereof, or the pharmaceutically acceptable salt thereof according to any one of claims 1 to 6, being a compound of formula (IIaa) or a tautomer, mesomer, racemate, enantiomer, diastereomer thereof, or mixture thereof, or a pharmaceutically acceptable salt thereof,

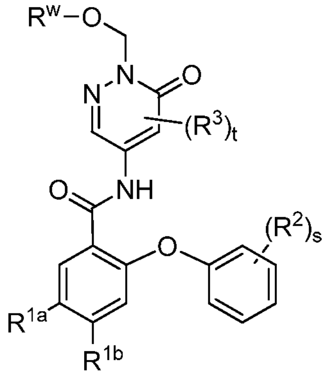

( IIaa )

wherein:

$R^{1a}$ is a halogen; and preferably, $R^{1a}$ is a Cl;
$R^{1b}$ is a haloalkyl; and preferably, $R^{1b}$ is a $CF_3$; and
$R^2$, $R^3$, $R^w$, s and t are as defined in claim 1.

**8.** The compound of formula (I) or the tautomer, mesomer, racemate, enantiomer, diastereomer thereof, or mixture thereof, or the pharmaceutically acceptable salt thereof according to any one of claims 1 to 6, wherein each $R^1$ is identical or different and each is independently selected from the group consisting of hydrogen atom, halogen, alkyl and haloalkyl; and preferably, each $R^1$ is identical or different and each is independently selected from the group consisting of hydrogen atom, halogen, $C_{1-6}$ alkyl and halo$C_{1-6}$ alkyl.

**9.** The compound of formula (I) or the tautomer, mesomer, racemate, enantiomer, diastereomer thereof, or mixture thereof, or the pharmaceutically acceptable salt thereof according to any one of claims 1 to 8, wherein each $R^2$ is identical or different and each is independently selected from the group consisting of hydrogen atom, halogen, alkyl, alkoxy, deuterated alkoxy, haloalkyl, haloalkoxy, cycloalkyl and cycloalkyloxy; and preferably, each $R^2$ is identical or different and each is independently selected from the group consisting of hydrogen atom, halogen, $C_{1-6}$ alkyl, $C_{1-6}$ alkoxy, halo$C_{1-6}$ alkyl, halo$C_{1-6}$ alkoxy, deuterated $C_{1-6}$ alkoxy and $C_{3-6}$ cycloalkyloxy.

**10.** The compound of formula (I) or the tautomer, mesomer, racemate, enantiomer, diastereomer thereof, or mixture thereof, or the pharmaceutically acceptable salt thereof according to any one of claims 1 to 9, wherein $R^3$ is a hydrogen atom.

**11.** The compound of formula (I) or the tautomer, mesomer, racemate, enantiomer, diastereomer thereof, or mixture thereof, or the pharmaceutically acceptable salt thereof according to any one of claims 1 to 10, being a compound of formula (III) or a tautomer, mesomer, racemate, enantiomer, diastereomer thereof, or mixture thereof, or a pharmaceutically acceptable salt thereof,

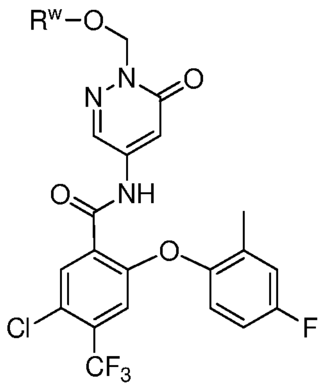

( III )

wherein:
$R^w$ is as defined in claim 1.

**12.** The compound of formula (I) or the tautomer, mesomer, racemate, enantiomer, diastereomer thereof, or mixture thereof, or the pharmaceutically acceptable salt thereof according to any one of claims 1 to 10, being a compound of formula (IV) or a tautomer, mesomer, racemate, enantiomer, diastereomer thereof, or mixture thereof, or a pharmaceutically acceptable salt thereof,

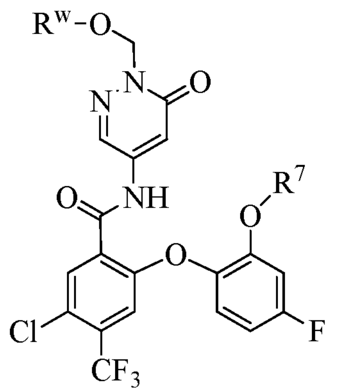

( IV )

wherein:

$R^7$ is selected from the group consisting of alkyl, deuterated alkyl and cycloalkyl; and preferably, $R^7$ is selected from the group consisting of $C_{1-6}$ alkyl, deuterated $C_{1-6}$ alkyl and $C_{3-6}$ cycloalkyl; and
$R^w$ is as defined in claim 1.

**13.** The compound of formula (I) or the tautomer, mesomer, racemate, enantiomer, diastereomer thereof, or mixture thereof, or the pharmaceutically acceptable salt thereof according to any one of claims 1 to 12, selected from the group consisting of:

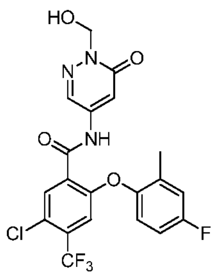
1

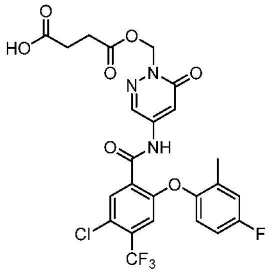
2

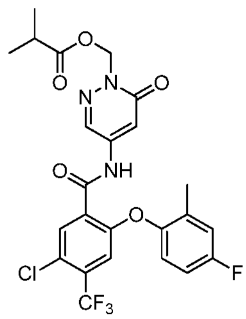
3

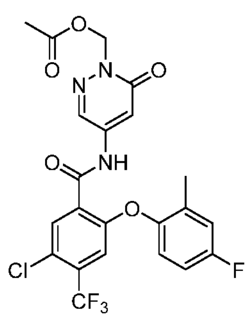
4

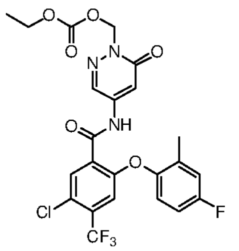
5

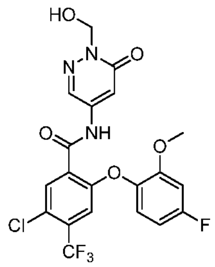
6d

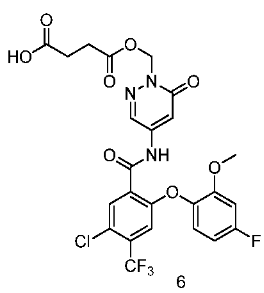
6

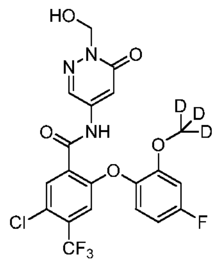
7e

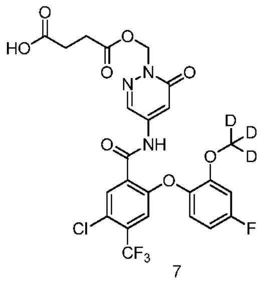
7

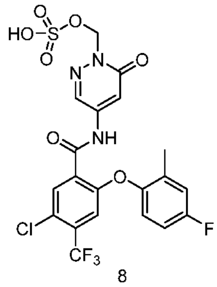
8

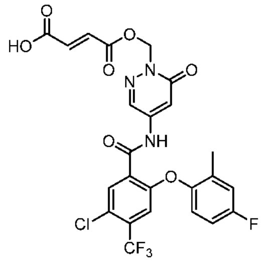
9

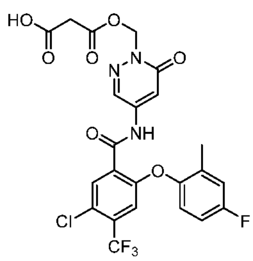
10

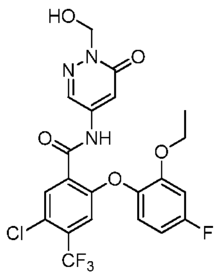

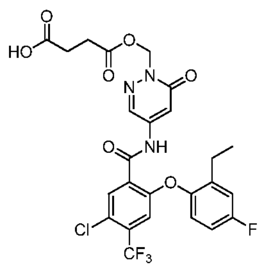
11

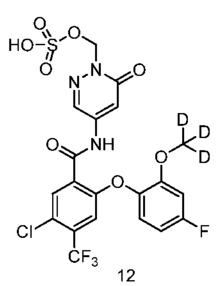
12

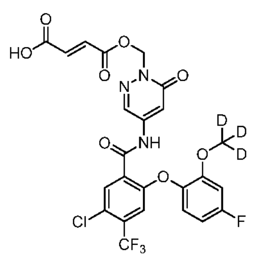
13

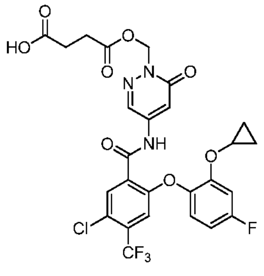

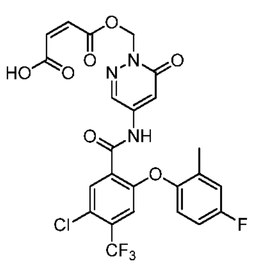

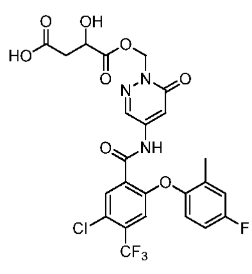

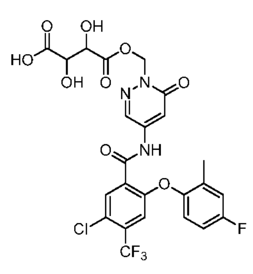

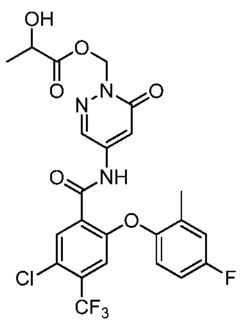
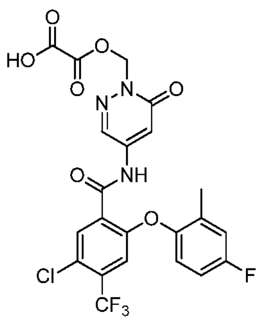
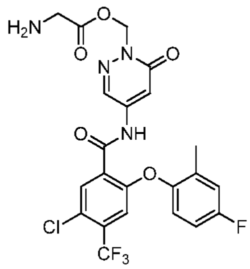
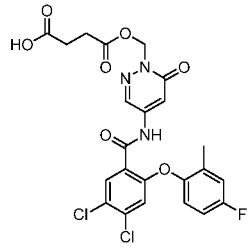

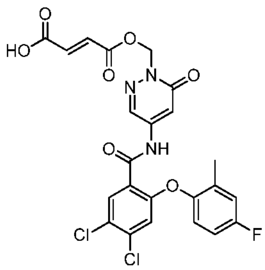
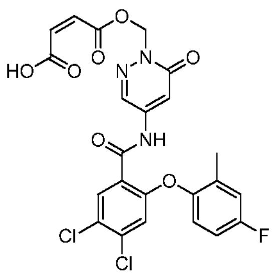
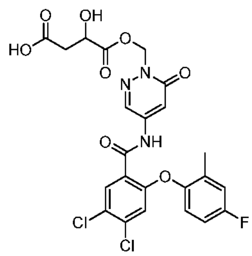
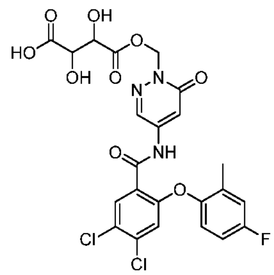

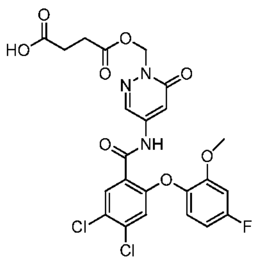
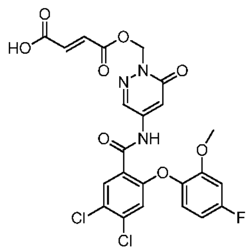
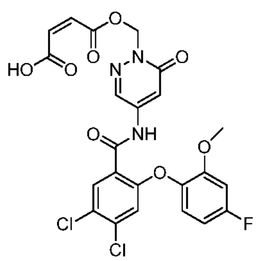
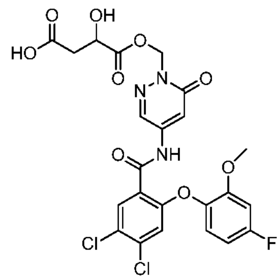

and

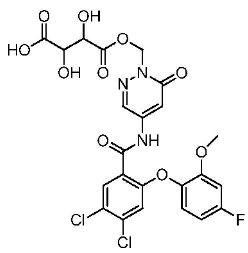

**14.** A method for preparing the compound of formula (I) or the tautomer, mesomer, racemate, enantiomer, diastereomer thereof, or mixture thereof, or the pharmaceutically acceptable salt thereof according to claim 1, comprising a step of:

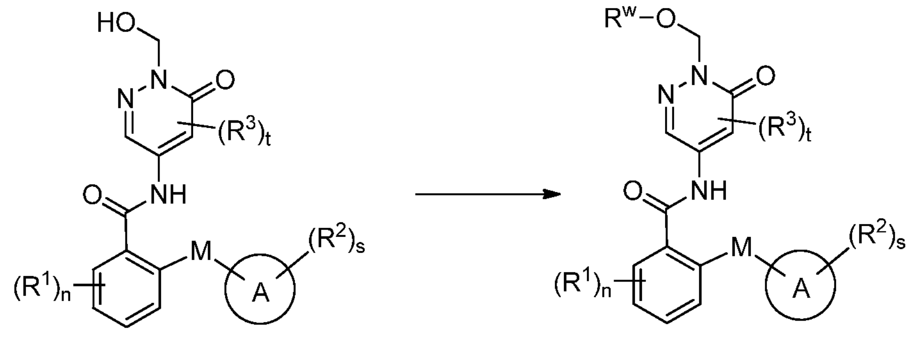

( IA ) ( I )

reacting a compound of formula (IA) with $R^w$-X,

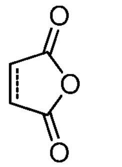

or sulfur trioxide pyridine to obtain the compound of formula (I);
wherein:

$R^w$ is $-C(O)R^6$ or $-S(O)_2OH$;
X is a halogen or hydroxy;
----- is a single bond or double bond; and
ring A, M, $R^1$, $R^2$, $R^3$, $R^6$, n, s and t are as defined in claim 1.

**15.** The method according to claim 14, further comprising a step of:

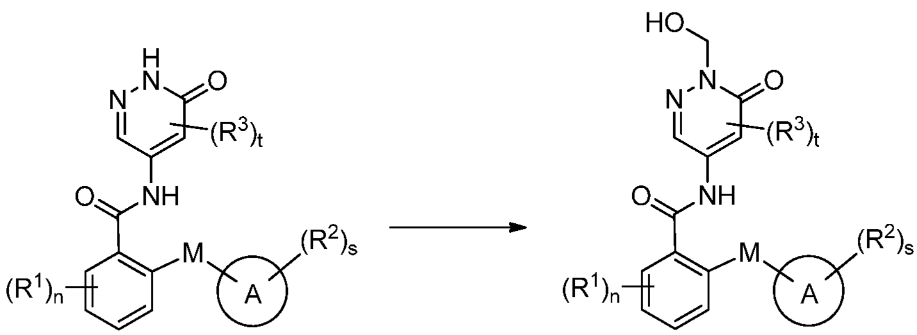

( IB ) ( IA )

reacting a compound of formula (IB) with formaldehyde solution to obtain the compound of formula (IA);
wherein:
ring A, M, $R^1$, $R^2$, $R^3$, n, s and t are as defined in claim 1.

**16.** A pharmaceutical composition, comprising the compound of formula (I) or the tautomer, mesomer, racemate, enantiomer, diastereomer thereof, or mixture thereof, or the pharmaceutically acceptable salt thereof according to any one of claims 1 to 13, and one or more pharmaceutically acceptable carriers, diluents or excipients.

**17.** Use of the compound of formula (I) or the tautomer, mesomer, racemate, enantiomer, diastereomer thereof, or mixture thereof, or the pharmaceutically acceptable salt thereof according to any one of claims 1 to 13 or the pharmaceutical composition according to claim 16 in the preparation of a medicament for inhibiting the voltage-gated sodium channel in a subject, wherein the voltage-gated sodium channel is preferaby $Na_v$ 1.8.

**18.** Use of the compound of formula (I) or the tautomer, mesomer, racemate, enantiomer, diastereomer thereof, or mixture thereof, or the pharmaceutically acceptable salt thereof according to any one of claims 1 to 13 or the pharmaceutical composition according to claim 16 in the preparation of a medicament for treating and/or alleviating pain and pain-related diseases, multiple sclerosis, Charcot-Marie-Tooth syndrome, incontinence or cardiac arrhythmia, wherein the pain is preferaby selected from the group consisting of chronic pain, acute pain, inflammatory pain, cancer pain, neuropathic pain, musculoskeletal pain, primary pain, intestinal pain and idiopathic pain.

**INTERNATIONAL SEARCH REPORT**

International application No.
**PCT/CN2020/075790**

**A. CLASSIFICATION OF SUBJECT MATTER**

C07D 237/14(2006.01)i； A61K 31/50(2006.01)i； A61P 29/00(2006.01)i

According to International Patent Classification (IPC) or to both national classification and IPC

**B. FIELDS SEARCHED**

Minimum documentation searched (classification system followed by classification symbols)

C07D; A61K; A61P

Documentation searched other than minimum documentation to the extent that such documents are included in the fields searched

Electronic data base consulted during the international search (name of data base and, where practicable, search terms used)

DWPI, CNABS(CN), CNTXT, WOTXT, USTXT, EPTXT, CNKI(CN), ISI-WEB OF SCIENCE, REGISTRY, CAPLUS: pyridazinone, 哒嗪酮, 疼痛, 止痛, pain, Analgesic, 电压门控钠, NaV, voltage-gated sodium, STN结构式检索, STN structured search, etc.

**C. DOCUMENTS CONSIDERED TO BE RELEVANT**

| Category* | Citation of document, with indication, where appropriate, of the relevant passages | Relevant to claim No. |
|---|---|---|
| A | WO 2014209094 A1 (UNIVERSITE MOHAMMED V SOUISSI) 31 December 2014 (2014-12-31)<br>see entire document, especially the abstract, and claim | 1-18 |
| A | CN 101238109 A (VERTEX PHARMACEUTICALS INCORPORATED) 06 August 2008 (2008-08-06)<br>see entire document, especially claims 1-60 | 1-18 |

☐ Further documents are listed in the continuation of Box C. ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **08 May 2020** | **21 May 2020** |
| Name and mailing address of the ISA/CN<br>**China National Intellectual Property Administration (ISA/CN)**<br>**No. 6, Xitucheng Road, Jimenqiao Haidian District, Beijing 100088**<br>**China** | Authorized officer |
| Facsimile No. **(86-10)62019451** | Telephone No. |

Form PCT/ISA/210 (second sheet) (January 2015)

**INTERNATIONAL SEARCH REPORT**
**Information on patent family members**

International application No.
**PCT/CN2020/075790**

| Patent document cited in search report | | | Publication date (day/month/year) | Patent family member(s) | | | Publication date (day/month/year) |
|---|---|---|---|---|---|---|---|
| WO | 2014209094 | A1 | 31 December 2014 | MA | 20150035 | A1 | 27 February 2015 |
| | | | | MA | 36055 | B1 | 31 December 2015 |
| CN | 101238109 | A | 06 August 2008 | US | 7989481 | B2 | 02 August 2011 |
| | | | | AU | 2006254809 | A1 | 14 December 2006 |
| | | | | NO | 20080141 | A | 09 January 2008 |
| | | | | US | 2009326023 | A1 | 31 December 2009 |
| | | | | ZA | 200800191 | A | 29 April 2009 |
| | | | | NO | 20080141 | B | 09 January 2008 |
| | | | | KR | 20080019693 | A | 04 March 2008 |
| | | | | ZA | 200800191 | B | 29 April 2009 |
| | | | | AU | 2006254809 | B2 | 15 March 2012 |
| | | | | US | 7605174 | B2 | 20 October 2009 |
| | | | | CN | 101238109 | B | 14 December 2011 |
| | | | | NZ | 564374 | A | 26 November 2010 |
| | | | | JP | 2012126745 | A | 05 July 2012 |
| | | | | JP | 2008543785 | A | 04 December 2008 |
| | | | | WO | 2006133459 | A1 | 14 December 2006 |
| | | | | MX | 2007015726 | A | 04 March 2008 |
| | | | | EP | 1888547 | A1 | 20 February 2008 |
| | | | | CA | 2611731 | A1 | 14 December 2006 |
| | | | | RU | 2007148504 | A | 27 June 2009 |
| | | | | HK | 1119433 | A1 | 05 October 2012 |
| | | | | IL | 187997 | D0 | 20 March 2008 |
| | | | | US | 2007117801 | A1 | 24 May 2007 |
| | | | | NO | 20080141 | L | 09 January 2008 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2016004417 A

### Non-patent literature cited in the description


- **RUSH A.M. et al.** *J. Physiol.,* 2007, vol. 579, 1-14
- **GOLDIN A.L. et al.** *Annu. Rev. Physiol.,* 2001, vol. 63, 871-894
- **FOZZARD H.A. et al.** *Physiol. Rev.,* 1996, vol. 76, 887-926
- **DIB-HAJJ S.D. et al.** *Annu. Rev. Neurosci.,* 2010, vol. 33, 325-347
- **SLEEPER A.A. et al.** *J.Neurosci.,* 2000, vol. 20, 7279-7289
- **YOSHIMURA N. et al.** *J.Neurosci.,* 2001, vol. 21, 8690-8696
- **TANAKA M. et al.** *G. NeuroReport,* 1998, vol. 9, 967-972
- **KERR B.J. et al.** *NeuroReport,* 2001, vol. 12, 3077-3080
- **FABER C.G. et al.** *Proc. Natl. Acad. Sci. USA,* 2012, vol. 109, 19444-19449